Europäisches Patentamt

(19) European Patent Office

Office européen des brevets

(11) EP 0 935 674 B1

(12) FASCICULE DE BREVET EUROPEEN

(45) Date de publication et mention
de la délivrance du brevet:
**10.08.2005 Bulletin 2005/32**

(51) Int Cl.⁷: **C12Q 1/68**

(86) Numéro de dépôt international:
**PCT/FR1997/001949**

(21) Numéro de dépôt: **97912288.4**

(22) Date de dépôt: **30.10.1997**

(87) Numéro de publication internationale:
**WO 1998/018959 (07.05.1998 Gazette 1998/18)**

(54) **PROCEDE DE POSITIONNEMENT DE CLONES PAR PEIGNAGE MOLECULAIRE**

VERFAHREN ZUR POSITIONIERUNG VON KLONEN MITTELS MOLEKULAREN KAEMMENS

METHOD FOR POSITIONING OF CLONES BY MOLECULAR COMBING

(84) Etats contractants désignés:
**AT BE CH DE DK ES FI FR GB GR IE IT LI LU MC NL PT SE**

(30) Priorité: **30.10.1996 FR 9613276**
**13.03.1997 FR 9702999**

(43) Date de publication de la demande:
**18.08.1999 Bulletin 1999/33**

(73) Titulaires:
• **INSTITUT PASTEUR**
**75015 Paris (FR)**
• **CENTRE NATIONAL DE**
**LA RECHERCHE SCIENTIFIQUE**
**75016 Paris Cédex (FR)**

(72) Inventeurs:
• **BENSIMON, Aaron**
**F-92160 Antony (FR)**
• **BENSIMON, David**
**F-75013 Paris (FR)**
• **MICHALET, Xavier**
**F-75018 Paris (FR)**

(74) Mandataire: **Warcoin, Jacques et al**
**Cabinet Régimbeau**
**20, rue de Chazelles**
**75847 Paris cedex 17 (FR)**

(56) Documents cités:
**WO-A-93/18186          WO-A-95/09929**
**WO-A-95/21939          WO-A-96/17958**
**WO-A-97/18326**

• **DESMAZE C ET AL: "IN SITU HYBRIDIZATION OF FLUORESCENT PROBES ON CHROMOSOMES, NUCLEI OR STRETCHED DNA: APPLICATIONS IN PHYSICAL MAPPING AND CHARACTERIZATION OF GENOMIC REARRANGEMENTS" CELLULAR AND MOLECULAR BIOLOGY, vol. 41, no. 7, novembre 1995, pages 925-931, XP000197050**
• **DU MANOIR ET AL.: "Quantitative analysis of comparative genomic hybridization" CYTOMETRY, vol. 19, 1995, pages 27-41, XP002036060**
• **BENSIMON ET AL.: "Alignement and sensitive detection of DNA by a moving interface" SCIENCE, vol. 265, 30 septembre 1995, pages 2096-2098, XP002036061**
• **PIPER ET AL.: "Computer image analysis of comparative genomic hybridization" CYTOMETRY, vol. 19, 1995, pages 10-26, XP002036062**
• **LICHTER P ET AL: "HIGH-RESOLUTION MAPPING OF HUMAN CHROMOSOME 11 BY IN SITU HYBRIDIZATION WITH COSMID CLONES" SCIENCE, vol. 247, no. 4938, 5 janvier 1990, pages 64-69, XP000371628**
• **MANOIR DU S ET AL: "DETECTION OF COMPLETE AND PARTIAL CHROMOSOME GAINS AND LOSSES BY COMPARATIVE GENOMIC IN SITU HYBRIDIZATION" HUMAN GENETICS, vol. 90, 1993, pages 590-610, XP000606241**
• **KALLIONIEMI A: "COMPARATIVE GENOMIC HYBRIDIZATION FOR MOLECULAR CYTOGENETIC ANALYSIS OF SOLID TUMORS" SCIENCE, vol. 258, no. 5083, 30 octobre 1992, pages 818-821, XP000353937**

**Description**

La présente invention concerne un procédé de positionnement de séquences polynucléotidiques (pouvant contenir ou non des gènes ou parties de gènes) dans un génome ou une partie de génome grâce à la technique dite du peignage moléculaire.

La présente invention concerne également un procédé de positionnement de réactifs d'origine biologique, naturelle ou synthétique, par association desdits réactifs avec tout ou partie de l'ADN peigné.

La technique du peignage moléculaire, telle que décrite dans les références ci-après : WO 95/22056 et WO 95/21939, appliquée aux acides nucléiques, et plus particulièrement à l'ADN génomique, permet l'extension uniforme et la visualisation de l'ADN ou de l'ARN sous forme de filaments rectilignes et pratiquement alignés.

La présente invention repose sur la mise en évidence du fait que, grâce à des sondes, c'est-à-dire des polynucléotides contenant un enchaînement de séquences nucléotidiques telles que des molécules d'ADN marquées qui reconnaissent spécifiquement des parties de l'ADN aligné, qu'on hybride sur l'ADN peigné, on peut directement visualiser sur le génome peigné la position de la séquence complémentaire.

Dans ces conditions, il est possible, par exemple en utilisant deux sondes marquées avec des chromophores différents tels que visualisables avec une coloration, rouge et vert par exemple, de mesurer la distance qui les sépare. Mais il est possible également, en utilisant différentes sondes ou une série de sondes contiguës (ci-après dénommées "contig"), de mesurer directement la longueur de la zone intéressante, et d'en mesurer les éventuelles altérations dans le cas d'un génome anormal.

La présente invention concerne un procédé de positionnement de plusieurs clones, tel que défini dans la revendication 1. Des méthodes pour le diagnostic des maladies génétiques sont également décrites. Ces méthodes, se caractérisant, de prérérence, par des altérations importantes du génome, soit dans sa structure, délétion, translocation par exemple, soit dans le nombre de copies de certaines séquences (trisomie par exemple où la séquence représente la totalité d'un chromosome), ainsi que des procédés permettant de localiser et de cartographier rapidement des gènes.

Le diagnostic génétique peut être divisé en plusieurs domaines:

- prénatal,
- pathologies à composante génétique,
- cancer et susceptibilité au cancer.

Diagnostic pré-natal

La majeure partie (95 %) des anomalies foetales sont ducs à des trisomies des chromosomes 21, 18, 13, X ou Y. Leur diagnostic indubitabic est plus moins tardif (17ème semaine d'aménorrhée, par amniocentèse par exemple). L'amniocentèse demande une ponction importante de liquide amniotique (quelques dizaines de millilitres) duquel des cellules foetales en suspension sont extraites et cultivées pendant plusieurs jours (voir la technique décrite par S. Mercier et J.L. Bresson (1995) Ann. Génét., 38, 151-157). Un caryotype de ces cellules est effectué par observation microscopique et comptage des chromosomes par un personnel très spécialisé.

Une technique faisant appel au prélèvement de villosités choriales permet de se dispenser de l'étape de mise en culture et évite le prélèvement de liquide amniotique. L'analyse du caryotype demande cependant le même travail (voir Médecine Prénatale. Biologie Clinique du Foetus. André Boué, ed. Flammarion, 1989). Ces deux techniques peuvent être appliquées plus précocement (jusqu'à 7 semaines de gestation pour le prélèvement de villosités choriales et 13-14 semaines pour l'amniocentèse), mais avec un risque légèrement accru d'avortement. Enfin, un prélèvement direct de sang foetal au niveau du cordon ombilical permet le caryotypage sans culture mais suppose une équipe de cliniciens spécialisés dans cette technique (Donner C. et al., 1996, Fetal Diagn, Ther., 10, 192-199).

D'autres anomalies comme des translocations ou des délétions/insertions de parties importantes de chromosomes peuvent être détectées à ce stade, ou en employant des techniques comme l'hybridation fluorescente in situ (FISH). Cependant, là encore, ce type de diagnostic ne peut être réalisé que par un personnel très qualifié.

Des études montrent, par ailleurs, qu'il n'existe pas encore de méthodes immunologiques permettant la détection de marqueurs foetaux dans le sang maternel permettant un diagnostic indubitable de la trisomie 21 ou d'autres anomalies (voir, par exemple, Wald N.J. et al., 1996, Br. J. Obstet. Gynaecol., 103, 407-412 pour le syndrome de Down lié à la trisomie 21).

Les diagnostics prénataux actuels ont donc de nombreux désavantages : ils ne peuvent être effectués qu'à un stade relativement tardif du développement de l'embryon; ils ne sont pas totalement dénués de risque pour le foetus ou pour la mère; les résultats sont souvent obtenus après un temps assez long (environ 1 à 3 semaines suivant la technique) et sont coûteux. Enfin, un certain nombre d'anomalies chromosomiques passent inaperçues.

Diagnostic de pathologies à composante génétique

De nombreuses maladies ont une composante génétique reconnue (diabète, hypertension, obésité, etc.) qui est le résultat de délétions, insertions et/ou remaniements chromosomiques de tailles variables. La mise en culture de cellules ne pose pas de problème à ce stade, mais les techniques de FISH, décrites par Lichter G.D. et al. (1993), Genomics, 16, 320-324 ; Brandritt B. et al. (1991), Genomics, 10, 75-82 et Van den Hengh G. et al. (1992), Science, 257, 1410-1412), ont une résolution limitée et font appel à un personnel très qualifié, rendant ces tests peu accessibles.

Le développement d'un test plus performant et bon marché permettrait l'adoption générale de thérapies adaptées, à un stade précoce des pathologies impliquées, susceptible d'améliorer leur rémission.

Diagnostic cancéreux

Parmi les pathologies à composante génétique, les affections cancéreuses constituent une classe importante affectant une partie croissante de la population. La compréhension actuelle du processus d'apparition d'un affection cancéreuse fait intervenir une étape de prolifération de proto-oncogènes (mutations dans le génome des cellules) qui précède la transformation de la cellule en cellule cancéreuse. Cette étape de prolifération n'est malheureusement pas détectable, alors que la possibilité d'effectuer un traitement à ce stade augmenterait assurément les chances de rémission et diminuerait le handicap des patients.

Enfin, nombre de tumeurs sont caractérisées par des remaniements chromosomiques tels que translocations, délétions, trisomies partielles ou complètes, etc.

Dans chacun de ces domaines, le peignage moléculaire peut apporter une contribution majeure, soit par la rapidité et la faible quantité de matériel biologique nécessaire, soit par la précision quantitative des résultats.

L'intérêt de la technique apparaît tout particulièrement dans le cas où le matériel génétique provient de cellules qui ne se divisent plus ou ne peuvent être mises en culture, voire de cellules mortes dans lesquelles l'ADN n'est pas significativement dégradé.

Dans le cas du diagnostic prénatal, il en est ainsi après extraction des cellules foetales circulant dans le sang maternel (Cheung et al., 1996, Nature Genetics 14, 264-268). Il en est de même dans le cas de cellules cancéreuses provenant de certaines tumeurs.

Le peignage moléculaire permet d'améliorer les possibilités de diagnostic de maladies génétiques, mais il peut également permettre l'étude et la mise en évidence des séquences génomiques responsables desdites maladies. D'ailleurs, actuellement, la mise au point d'un "kit" ou coffret de diagnostic commence par la recherche du gène impliqué dans la pathologie.

La recherche de gènes impliqués dans des pathologies (humaines ou autres) est aujourd'hui effectuée en général en plusieurs étapes :

(i) Etablissement d'une population cible d'individus atteints par la pathologies, de leurs descendants, ascendants et collatéraux, et prélèvement d'échantillon sanguins et/ou cellulaires en vue du stockage de matériel génétique (sous forme d'ADN ou de souches cellulaires).

(ii) Localisation génétique par analyse de probabilité de co-ségrésation avec des marqueurs génétiques (linkage analysis). A ce stade de l'étude, on dispose de quelques marqueurs proches localisés sur un (ou plusieurs) chromosome(s) donné(s) qui permettent de passer à l'étape de localisation physique.

(iii) Cartographie physique : à partir des marqueurs génétiques obtenus à l'étape précédente, un criblage de banques de clones d'ADN humain (YACs, BACS, cosmides ou autres) spécifiques de la ou des régions déterminées à l'étape précédente est effectué. On obtient ainsi un certain nombre de clones contenant les marqueurs précédents. La zone d'intérêt peut être alors cartographiée précisément en utilisant des clones de taille décroissante. Un clonage de la partie du génome considérée peut également être effectuée à nouveau à partir de l'ADN humain.

(iv) Recherche du gène : plusieurs techniques peuvent être utilisées à ce stade: exon "trapping" (utilisation de librairies de cDNA (ADN coupplémentaires obtenus à partir d'ARN messager)), ilôts CpG, conservation de séquences inter-spécifiques, etc, qui permettent d'assigner une séquence codante à l'un (ou plusieurs) des clones sélectionnés aux étapes précédentes.

L'ensemble de cette stratégie représente un travail important (jusqu'à plusieurs années, éventuellement). Aussi, toute technique permettant d'arriver plus rapidement à l'étape (iv) constitue un atout pour la recherche de gènes mais également dans le diagnostic.

Dans l'état actuel de la technique, lorsque le gène a été localisé, par exemple par la méthode précédente, sa détection est en général effectuée grâce à des sondes spécifiques correspondant à la séquence en cause, celle-ci étant amplifiée par des méthodes de type PCR par exemple ou de type LCR (telle que décrite dans le brevet EP 0 439 182) ou une technique de type NASBA (kit commercialisé par la société Organon Teknika).

Toutefois, les techniques d'amplification ne sont pas totalement satisfaisantes, notamment pour les hétérozygotes, puisqu'il existe une copie normale du gène dans le génome, de même que dans le cas de grande délétion ou de maladies à séquences répétées où la PCR n'est pas non plus satisfaisante.

Le diagnostic d'un grand nombre de maladies génétiques peut être maintenant envisagé en mettant en oeuvre le peignage moléculaire et le marquage de l'ADN. Desmaze et Aurias, Cellular and Molecular Biology, 1995 décrivent une technique FISH appliquée à la cartographie et la caractérisation de réarrangements génomiques par une technique d'étirement de chromatine. Cette technique consiste à étirer une fibre d'ADN contenant plusieurs molécules, ce qui nuit de façon notable à sa sensibilité.

Le peignage moléculaire est une technique consistant à ancrer des molécules d'ADN par leurs extrémités sur des surfaces dans des conditions physico-chimiques bien définies, suivi de leur étirement à l'aide d'un ménisque en récession ; on obtient ainsi des molécules d'ADN alignées parallèlement. L'ADN purifié utilisé peut être de toute taille, et donc notamment de l'ADN génomique extrait de cellules humaines. Le génome peut également provenir d'un matériel génomique comportant au moins 80 % de matériel génétique d'origine foetale.

Les molécules d'ADN ainsi peignées peuvent être dénaturées avant d'être hybridées avec des sondes d'acides nucléiques marquées par tout moyen approprié, (notamment avec des nucléotides biotine-dUTP, ou digoxygénine-dUTP), lesquelles sont alors révélées, par exemple à l'aide de systèmes d'anticorps fluorescents.

Le peignage moléculaire se caractérisant par une extension constante des molécules peignées, la mesure des longueurs des fragments fluorescents observés à l'aide d'un microscope à épifluorescence (par exemple) donne donc directement la taille des fragments de sondes hybridés.

Le taux d'extension dépend du type de surface, mais peut être précisément mesuré, il est par exemple de 2 kilobases (kb) par micromètre (µm) dans le cas de surfaces silanisées suivant le protocole décrit dans la référence (1) et mis en oeuvre dans les exemples.

Lorsque cela est nécessaire, il est possible de prévoir un standard interne, c'est-à-dire un ADN de longueur connue dit de calibrage, qui permettra d'étalonner la manipulation, c'est-à-dire de calibrer chaque mesure.

La présente invention, est définie par les revendications 1-8 et concerne un procédé, caractérisé en ce que

    (a) on fixe et on peigne une certaine quantité dudit génome sur une surface de peignage,
    (b) on hybride le produit de peignage avec des sondes marquées avec des éléments radioactifs, fluorescents ou autres, tels que billes, particules, correspondant à chaque clone, de sorte que lesdites sondes pourront être révélées spécifiquement, par une couleur notamment,
    (c) on prélève l'information correspondant à la place de chaque clone ainsi que les tailles et les distances correspondantes sur le génome,
    (d) on réitère les opérations b) et c) n fois en modifiant la couleur, le marquage ou le mode de révélation des sondes, sachant qu'avec pcouleurs, marquages ou modes de révélation différents, au bout de n hybridations on peut positionner $p^n$ clones.

Dans le cadre des méthodes standards de cartographie, le nombre 1 d'hybridations nécessaires pour cartographier N clones à l'aide de p marquages, couleurs ou modes de révélation, croît linéairement avec le nombre de clones N.

Ainsi, avec 3 couleurs, il est aujourd'hui nécessaire d'effectuer au moins 15 hybridations du type précédent pour cartographier 30 clones.

Ce nombre d'hybridation est important, et le nombre de couleurs disponibles est en pratique limité (même en utilisant des combinaisons de fluorophores). Par ailleurs, une fois la totalité des mesures accomplies, il faut procéder à la sélection des différentes positions possibles des clones, ce qui peut ne pas être toujours aisé, si l'on tient compte des incertitudes de mesure.

La méthode proposée ici permet de cartographier un nombre de clone croissant exponentiellement avec le nombre d'hybridations effectuées.

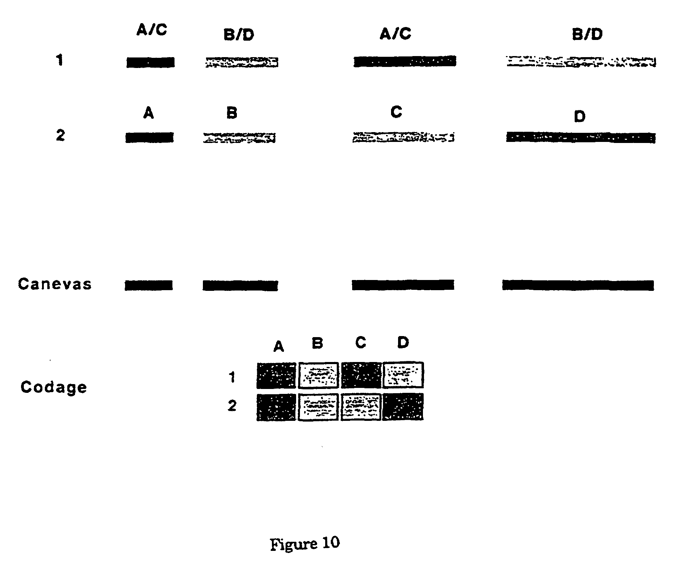

A titre d'illustration, le schéma de la figure 10 représente le résultat de deux hybridations de 4 clones révélés avec 2 couleurs différentes d'une hybridation à l'autre (pour la moitié d'entre eux). Les 4 clones hybridés forment un canevas coloré de façon différente d'une hybridation à l'autre, les clones étant repérable via leur codage (binaire dans ce cas). Dans cet exemple de 4 clones, il suffit d'un code composé d'une succession de 2 couleurs pour distinguer chaque clone :

A = Rouge puis Rouge

B = Vert puis Vert

C = Rouge puis Vert

D = Vert puis Rouge

De 5 à 8 clones, 3 hybridations seront necessaires, pour distinguer les clones par une succession de 3 couleurs.

Plus généralement, à l'aide de p couleurs, pour cartographier N clones, il suffira d'un nombre d'hybridations I tel que : $N = p^I$

Par comparaison avec la méthode standard, 30 clones pourront être cartographiés en 5 hybridations (au lieu de 30) si l'on ne dispose que de 2 couleurs, et en seulement 4 hybridations (au lieu de 15) si l'on dispose de 3 couleurs.

Le principe de cartographie présenté ici est simple. Cependant, afin de surmonter certains artefacts expérimentaux possibles (dispersion de tailles des signaux, variabilité de la saturation, cassure des molécules, etc.), l'on utilisera avec avantage un logiciel de traitement d'images et d'analyse statistique adéquat.

Les exemples ci-après permettront de mieux comprendre d'autres caractéristiques et avantages de la présente invention.

Enfin, dans un quatrième mode de mise en oeuvre, la présente invention concerne un procédé permettant notamment la détection ou le positionnement de produits susceptibles de réagir avec l'ADN peigné. Par exemple, des protéines de régulation de la transcription de gène se liant à l'ADN au cours du cycle cellulaire ou non pourront être détectées sur ADN peigné, et leurs sites de liaison préférentielle déterminés par rapport à la position de séquences connues et repérées, par exemple, suivant le procédé précédent de mise en oeuvre de l'invention.

De façon analogue, des molécules d'intérêt thérapeutique susceptibles de réagir avec l'ADN pourront être détectées sur ADN peigné ; leur effet sur d'autres molécules susceptibles de réagir avec l'ADN pourrait également être étudié par comparaison.

Parmi les molécules susceptibles de réagir avec l'ADN peigné, il faut citer les protéines de régulation telles que décrites par :

- Laughon et Matthew (1984), Nature, 310 : 25-30 pour les protéines de régulation s'attachant sur de l'ADN de drosophile,
- Struhl K. et al. (1987), Cell, 50 : 841-846 pour des protéines de régulation se liant à l'ADN dans leur domaine de liaison spécifique (« binding-domain »).

Ces molécules peuvent également être des intercalants ou des molécules modifiant l'ADN comme décrits par :

- Echols H. et al. (1996), Science, 223 : 1050-1056 sur les multiples interactions de l'ADN induisant, par exemple, des transcriptions ;
- dans la revue An. Rev. of Bioch. (1988), 57,: 159-167, Gross et Ganard décrivent l'hypersensibilité des sites nucléases dans la chromatine ;
- Hanson et al. (1976), Science, 193: 62-64 décrivent le psoralène comme agent photoactif dans le clivage sélectif des séquences nucléotidiques :
- Cartwight et al. (1984), NAS,10 : 5835-5852.

Dans la présente description, la technologie de peignage fait référence à la technologie décrite dans les documents mentionnés précédemment, de même que la notion de "surface de peignage" qui correspond à une surface traitée permettant l'ancrage de l'ADN et son étirement par un ménisque en récession.

Il faut noter que la surface de peignage est, de préférence, une surface plane sur laquelle les lectures sont plus commodes.

Par "réaction entre les sondes marquées et l'ADN peigné", on entend toute réaction chimique ou biochimique, en particulier des réactions de type immunologique (par exemple anticorps dirigé contre de l'ADN méthylé), des réactions protéines/ADN ou acides nucléiques/ADN (par exemple l'hybridation entre segments complémentaires) ou acides nuctéiques/ARN, ou acides nucléiques/hybrides ARN-ADN. On citera également pour exemple les réactions de liaison chimique ADN-ADN utilisant des molécules de psoralène ou les réactions de polymérisation d'ADN à l'aide d'une enzyme polymérase.

L'hybridation est, en général, précédée d'une dénaturation de l'ADN fixé et peigné, cette technique est connue et ne sera pas décrite en détail.

Par "sonde", on entend aussi bien désigner un polynucléotide mono ou bicaténaire, comportant au moins 20 nucléotides de synthèse ou un fragment d'ADN génomique, qu'un "contig", c'est-à-dire un ensemble de sondes qui sont contiguës ou chevauchantes et recouvre la zone en cause, ou plusieurs sondes séparées, marquées ou non. On entend également par "sonde" toute molécule liée de façon covalente ou non à l'une au moins des entités précédentes, ou toute molécule biologique, naturelle ou synthétique, pouvant réagir avec l'ADN, le sens donné au terme "réaction" ayant été précisé ci-dessus, ou toute molécule liée de façon covalente ou non à toute molécule pouvant réagir avec l'ADN.

Dans le cadre de l'invention, les sondes sont marquées . Ainsi, dans le cas où les sondes seraient marquées par des cytosines méthylées, elles pourraient être révélées, après réaction avec le produit du peignage, par des anticorps fluorescents dirigés contre ces cytosines méthylées. Les éléments assurant le marquage pourront être radioactifs mais seront, de préférence, des marquages froids, par fluorescence par exemple. Il peut également s'agir de sondes nucléotidiques dont certains atomes sont remplacés.

La taille des sondes pourra s'entendre de toute valeur mesurée avec une unité extensive, c'est-à-dire telle que la taille de deux sondes égale la somme des tailles des sondes prises separement. Un exemple est donné par la longueur, mais une intensité de fluorescence pourra par exemple être utilisée. La longueur des sondes utilisées est comprise, par exemple, entre 5 kb et 40-50 kb, mais peut également être constituée par la totalité du génome peigné

De préférence, la réaction de la sonde avec l'ADN peigné est modulée par une ou plusieurs molécules, solvants ou autres paramètres pertinents.

Enfin, de façon générale, dans ce qui va suivre on parlera de "génome", il faut bien entendre qu'il s'agit d'une simplification, toute séquence d'ADN ou d'acides nucléiques susceptible d'être fixée sur une surface de peignage est englobée dans cette terminologie.

En outre, le terme de "gène" sera parfois utilisé indifférem-ment pour désigner une "partie de gène" d'origine génomique ou bien une "séquence polynucléotidique" synthétique spécifique.

Un procédé décrit dans la demande mais ne faisant pas partie de l'invention, est utilisé pour permettre le diagnostic de cassures dans un génome, de même que pour le clonage positionnel de telles cassures. Il convient de remarquer que le terme "cassure" recouvre un grand nombre de modifications locales du génome dont la liste sera explicitée plus loin.

Le procédé consiste à déterminer la position des éventuels points de cassure impliqués dans une pathologie d'origine génétique par hybridation sur ADN génomique peigné de patients atteints de la dite pathologie, d'une sonde génomique de taille connue (clonée ou autre) située dans la région du gène recherché. Ces points de cassure consistent en points dans la séquence génétique dont l'environnement change sur plusieurs kilobases (kb) entre individu sain et individu malade.

Le principe de définition du point de cassure repose sur la possibilité de détecter par peignage moléculaire une modification locale du génome étudié par rapport à un génome déjà étudié, au niveau de la ou des régions considérées.

La mise au point de méthodes de repérage de modifications locales du génome de tailles inférieures à 1 kb est ainsi envisageable à l'aide de techniques d'observation en champ proche (AFM, STM, SNOM, etc.) ou possédant une résolution intrinsèquement supérieure (par exemple, microscopie électronique de nano billes d'or).

Plus particulièrement, la demande décrit un procédé de mise en évidence d'une anomalie génétique de cassure dans un génome, caractérisé en ce que:

(a) on fixe et on peigne une certaine quantité dudit génome sur une surface de peignage,

(b) on hybride le produit de peignage avec une ou plusieurs sondes spécifiques marquées correspondant à la séquence genomique dont on cherche l'anomalie,

(c) on mesure la taille des fragments correspondant aux signaux d'hybridations et éventuellement leur répétition, et

(d) on en déduit la présence d'une cassure, soit par mesure directe soit par comparaison avec un étalon correspondant à une longueur témoin.

A titre d'illustration, la mesure de la taille des fragments conduit à un histogramme, c'est-à-dire une représentation graphique des longueurs des fragments observés.

Afin de réaliser un histogramme de la sonde, on évalue le nombre de clones présentant une longueur de sonde déterminée. En principe l'histogramme ne comporte qu'un ou deux pics suivant le type de cassure analysée, deux pics lorsque la sonde s'hybride en deux fragments séparés et un seul pic lorsqu'elle s'hybride en un seul fragment.

Dans le cas d'un génome hétérozygote, dont l'un des allèles est normal pour la région considérée, la signature de l'allèle normal (l'absence de cassure) se superpose à celle de l'allèle anormal, mais peut être extraite du fait qu'elle est connue.

Ce procédé peut être également utilisé pour effectuer du clonage positionnel, c'est-à-dire pour déterminer la position d'un ou plusieurs gènes inconnus impliqués dans une pathologie. Le principe consiste, comme précédemment, à hybrider des clones d'ADN humain, ou animal ou végétal, servant alors de sonde sur l'ADN génomique peigné d'un ou de plusieurs patients atteints de la pathologie étudiée. La révélation de ces hybridations permet de mesurer la taille des fragments hybridés et de construire un histogramme des différentes tailles observées. Si le clone utilisé comme sonde recouvre un point de cassure du gène, la signature de ce phénomène sera lisible sur la longueur (plus petite) du fragment hybridé.

L'utilisation d'un nombre limité de clones spécifiques de la région incriminée qui pourra avoir été déduite par analyse de liaison génétique ("linkage analysis"), permettra ainsi une détermination rapide et précise de la position d'un point de cassure, d'une délétion ou de tout autre remaniement génétique de taille suffisante pour être résolue par la technique de détection associée au peignage moléculaire.

Dans ce cas évidement, on recherche la cassure afin de la cartographier, dans le diagnostic la cassure est connue, c'est sa présence ou son absence qui est recherchée.

Deux cas de figure peuvent se présenter (dans l'hypothèse où il existe un point de cassure dans la région du génome impliquée dans le pathologie):

(i) la sonde ne chevauche pas le point de cassure,
(ii) la sonde chevauche le point de cassure.

Dans le cas (i), la mesure des longueurs des sondes fluorescentes est comparable à celle qui serait obtenue avec la même sonde hybridée sur un ADN génomique non pathologique de même nature (c'est-à-dire essentiellement, de même taille et préparé dans les mêmes conditions).

Dans le cas (ii), au contraire, la sonde étant systématiquement hybridée sur deux morceaux (ou davantage) séparés dans l'ADN génomique peigné (par définition de l'existence d'un point de cassure), la mesure des longueurs des sondes fluorescentes hybridées se distingue du résultat obtenu par hybridation sur un ADN génomique non pathologique. De surcroît, la taille des fragments hybridés sur l'ADN pathologique permet d'estimer la position du point de cassure au sein du clone avec une précision de quelques kb, voire davantage, si une technique plus résolutive est utilisée.

De ce fait, il ne reste alors plus qu'à rechercher le gène dans ce clone. Pratiquement, il s'agira de répéter ces mesures pour l'ensemble des clones susceptibles de recouvrir partiellement la zone correspondant au gène. Le nombre de lames d'hybridations pourra être réduit en hybridant simultanément plusieurs sondes marquées différemment, ou en utilisant une méthode de codage par combinaison de couleurs, comme cela sera décrit ci-après.

Cette technique permet de déterminer la position des éventuels points de cassure de la région du génome impliquée dans une pathologie génétique par hybridation d'ADN génomique cloné sur de l'ADN génomique peigné provenant de patients. Cette technique s'applique donc à la recherche de régions du génome responsables de pathologies dues à:

- la délétion d'une partie ou de la totalité de cette région du génome,

- la translocation de toute ou partie de cette région du génome,
- la duplication ou présence de plusieurs copies de toute ou partie de cette région du génome en son sein ou en tout autre endroit du génome,
- l'insertion de toute séquence génétique à l'intérieur de cette région du génome.

Dans un second cas, et dans certains cas particuliers, notamment lorsque l'anomalie génétique recherchée comporte des délétions importantes ou des duplications (cas des trisomies par exemple), un procédé décrit dans la présente demande peut être modifié puisqu'il s'agit alors de doser les gènes ou une séquence particulière.

Plus particulièrement, il est décrit un procédé de dosage d'une séquence génomique déterminée dans un génome, caractérisé en ce que :

(a) on fixe et on peigne une certaine quantité dudit génome sur une surface de peignage,
(b) on hybride le produit de peignage avec une sonde témoin marquée de longueur lt correspondant à une séquence génomique dite témoin, c'est-à-dire dont on connaît le nombre de copies dans le génome, et avec une sonde spécifique marquée de longueur le correspondant à la séquence génomique à doser, de sorte que lesdites sondes pourront être identifiées séparément,
(c) on mesure alors la longueur totale des signaux d'hybridation pour les deux sondes, soit Lc et Lt,
(d) on calcule pour chacune le nombre de copies de la séquence correspondant par le rapport

$$Nt = \frac{Lt}{lt} \quad et \quad Nc = \frac{Lc}{lc}$$

et on en déduit le nombre de copies de la séquence à doser par rapport à la séquence témoin.

Dans le cas du diagnostic prénatal de la trisomie 21, la méthode pourra consister en l'hybridation d'une sonde cosmidique spécifique d'un chromosome témoin (chromosome 1, par exemple- sonde de longueur lt) marquée avec des nucléotides biotinilés, et l'hybridation d'une sonde cosmidique spécifique du chromosome 21 (sonde de longueur lc) marquée à la digoxygénine sur de l'ADN génomique peigné extrait de prélèvements amniotiques, ou de tout autre prélèvement contenant des cellules d'origine foetale.

Par exemple, on pourra utiliser un système de révélation avidine-Texas Red (couleur rouge) pour la sonde témoin et antidigoxygénine-FITC (couleur verte) pour la sonde spécifique: la longueur totale des signaux d'hybridation rouges observés dans une zone donnée de la surface, LT, et la longueur totale des signaux d'hybridation verts observés dans la même zone, ou dans une zone équivalente de la surface, LC, conduisent donc aux nombres Nt et Nc définis ci-dessus.

Le rapport Nc/Nt proche de 1 signalera un génotype normal (2 chromosomes 21 pour 2 chromosomes 1), alors qu'un rapport proche de 1,5 signalera un génotype trisomique (3 chromosomes 21 pour 2 chromosomes 1).

De façon générale, une différence significative entre Nc et la valeur attendue pour le nombre de génomes présents déduit de Nt est l'indication de la présence d'une anomalie génique.

Dans le cas du diagnostic d'oncogènes ou proto-oncogènes, la même méthode pourra être employée: une sonde témoin sera hybridée et révélée en rouge par exemple et une sonde correspondant au gène ou à une partie du gène recherché sera hybridée et révélée en vert par exemple. Après les mesures effectuées comme ci-dessus, le rapport Nc/Nt donnera l'abondance relative du gène par rapport à la fréquence de deux copies par génome diploïde.

La méthylation aberrante des îlots GpC observée fréquemment dans de nombreux cancers (92% des cancers du colon) peut aussi être détectée par le procédé selon l'invention par réaction entre l'ADN peigné et des anticorps fluorescents dirigés contre les cytosines méthylées.

En effet, la perte de l'hétérozygotie sur le chromosome 9p21 est l'une des altérations génétiques les plus fréquentes identifiées dans les cancers humains. Le gène suppresseur de tumeur CDKN2/p16/MTS1 localisé dans cette région est fréquemment inactivé dans beaucoup de cancers humains par délétion homozygote. Toutefois, on a rapporté un autre mode d'inactivation qui implique la perte de la transcription associée avec une méthylation *de novo* des îlots 5' GpC de CDKN2/p16 dans les cancers des poumons, les gliomes et les carcinomes à desquamation de la tête et du cou. Ces méthylations aberrantes des îlots GpC arrivent également fréquemment dans des lignées cellulaires de cancers du sein (33%), de la prostate (60%), du rein (23%) et du colon (92%) (Herman J.G. et al. (1995) Cancer Res., Oct. 15, 55(20) : 4525-30 ; Wales M.M. et al. (1995) Nature Med., Jun., 1 (6) : 570-607).

La localisation précise des aires de méthylation sur un gène est d'une très grande importance pour la compréhension du mécanisme du développement du cancer et pour un test de "screening" possible. Le peignage moléculaire peut détecter avec une précision de quelques kb la localisation de tels îlots GpC impliqués dans le développement du cancer.

Cette technique permettant de déterminer le nombre de copies d'un gène dans un génome peut éventuellement être utilisée pour détecter l'absence d'une partie du génome.

Dans le cas d'une pathologie caractérisée par la délétion d'une partie importante d'un chromosome, il suffit en effet de prendre pour séquence cible un clone contenu dans la zone délétée, et pour séquence témoin un clone extérieur à cette zone. Il est de la sorte possible de détecter des délétions de la taille d'un clone cosmidique (30-50 kb) ou supérieure.

Si l'on dispose d'une densité de molécules peignées suffisante, il est envisageable de détecter des délétion plus petites (quelques kb), correspondant à une partie de la séquence cible utilisée. C'est la raison pour laquelle il est particulièrement intéressant de disposer, sur la surface peignée, au moins une dizaine de copies de génome.

L'incertitude statistique sur le rapport Nc/Nt est d'ordre $1/\sqrt{Nc} + 1/\sqrt{Nt}$. Avantageusement, il convient de disposer sur la surface peignée d'un nombre suffisant de signaux pour avoir une incertitude statistique de moins de 20 % sur le rapport Nc/Nt. Il importe donc d'avoir un grande nombre de sondes hybridées, typiquement Nc, Nt > 100.

Cependant, dans la pratique, il est aussi possible d'augmenter la précision de ces mesures en utilisant, non pas un mais plusieurs types de sondes témoins et de sondes cibles, sans nécessairement chercher à distinguer entre ces types de sondes, c'est-à-dire en les révélant toutes de la même façon.

La possibilité d'obtenir un tel nombre de signaux a été démontrée: il est possible de recenser une centaine de signaux sur une surface de verre silanisée de 20 x 20 mm de surface utile. Cette densité pourra être considérablement augmentée dès lors qu'on dispose d'une quantité importante d'ADN.

Il apparaît qu'un nombre suffisant de génome par surfaces de 20 x 20 mm d'aire utile se situe aux alentours de 100, lorqu'une seule sonde est utilisée. Dans le cas de l'utilisation de plusieurs sondes, ou de sondes plus grandes, il est envisageable de pouvoir réduire soit:

- la surface de peignage et donc la surface analysée.
- la densité d'ADN utilisée, donc le nombre de génomes peignés en surface.

Suivant la contrainte principale (rapidité exigée, ou ADN en quantité limitée), l'une ou l'autre de ces deux voies pourra être utilisée.

La technique exposée implique l'utilisation de protocoles de préparation stricts mais sans difficultés techniques particulières. Au niveau de l'analyse des signaux, aucune qualification particulière n'est nécessaire, rendant de la sorte la technique généralisable à tous les laboratoires possédant un personnel avec des compétences minimales en biologie moléculaire.

Quelques centaines de milliers de cellules devraient en principe suffire pour préparer une solution d'ADN génomique conduisant à une densité importante de molécules peignées sur les surfaces d'analyse. Il n'est donc plus en principe nécessaire de réaliser de cultures cellulaires dans la plupart des cas. L'ensemble prélèvement-analyse devrait donc pouvoir être réalisé en quelques jours.

La simplicité des signaux à analyser (parallèles et distincts du bruit de fond d'hybridation) permet d'envisager une automatisation complète du processus d'analyse des signaux (scan des surfaces, acquisition et exploitation des mesures). L'intégration avec un système de stockage de surfaces correspondant à différents patients permet d'envisager des rendements important, donnant la possibilité de fournir divers types de diagnostics en quelques jours.

Le procédé décrit précédemment peut permettre différents types de diagnostics: comptage de chromosome (trisomie, monosomie, etc.), comptage des exemplaires d'un gène, détection de délétions connues, ou autres modifications chromosomiques se traduisant par une modification de la longueur hybridée d'une sonde genomique donnée par génome.

Il est à noter qu'il est aussi possible de détecter une délétion partielle sur un seul allèle.

Il est pareillement possible d'effectuer l'hybridation de clones sur plusieurs génomes différents peignés sur une même surface. Par exemple, le peignage simultané du génome d'un organisme principal et du génome d'organismes hôtes (parasites, bactéries, virus ...) et l'utilisation de sondes spécifiques, d'une part de l'organisme principal, et, d'autre part des organismes hôtes, permet en principe de déterminer le rapport nombre d'hôtes/nombre de cellules de l'organisme principal. Dans le cas d'un organisme infecté par un virus, cela permet la mesure de la charge virale. Les chiffres cités plus haut limitent probablement la sensibilité de cette méthode de diagnostic à des situations où l'on trouve plus d'un organisme infectieux pour 100 cellules hôtes environ.

Ces différents types de diagnostics peuvent être combinés grâce à l'utilisation de systèmes de révélation multiples (plusieurs couleurs, ou combinaison de couleurs), ou tout autre méthode permettant la distinction entre les signaux d'hybridation provenant de sondes distinctes et destinées à un diagnostic précis.

Le principe de la technique reposant sur le peignage de l'ADN du patient, cette étape de préparation de l'ADN nécessite des protocoles d'extraction, peignage et le matériel correspondant (surfaces traitées, appareil de peignage moléculaire).

Le diagnostic lui-même nécessite l'hybridation de sondes nucléotidiques spécifiques et la révélation de ces sondes, par exemple par des systèmes d'anticorps. Un codage par couleur pouvant en outre être effectué dans le cas de diagnostics combinés, il est donc possible de proposer des lots de sondes pré-marquées correspondant à un catalogue de diagnostics particuliers.

Est également decrit un procédé permettant notamment la cartographie physique d'un génome.

Le but de la cartographie physique étant l'ordonnancement de clone au sein d'un génome, le peignage moléculaire s'applique naturellement à cet objectif, par simple hybridation des clones sur le génome peigné (par exemple, dans le cas d'un YAC, le génome entier de la levure peut être peigné, pour faire l'économie de la séparation du chromosome artificiel des chromosomes naturels de la levure).

La position des clones est obtenue par mesure directe de leur distance à un clone de référence, ou à tout autre signal d'hybridation de référence sur le génome peigné. L'extension constante de l'ADN peigné permet alors d'établir directement en kilobases (kb) la position respective des clones ainsi que leur taille, lorsque celle-ci dépasse la résolution de la méthode. Notamment, dans le cas de la microscopie classique par épifluorescence, dont la résolution est d'une demi-longueur d'onde, la cartographie précise d'ADNc (ADN complémentaire des ARN transcrits dans la cellule) est possible, mais sans possibilité de mesure précise de la taille des fragments hybridés (exons), qui est de l'ordre de quelques centaines de bases en général. Cependant, par cette méthode, la localisation précise au sein de l'ADN génomique de fragments d'ADNc complets ou de leurs fragments peut être obtenue. Par exemple, on peut déterminer la présence ou l'absence et la position des ADNc correspondant à une protéine d'intérêt par hybridation des ADNc sur l'ADN génomique ou sur un clone d'ADN génomique (cosmide, BAC, YAC, par exemple) en même temps qu'un clone servant de repère.

L'utilisation de multiples fragments obtenus à partir d'un ADNc conduit au repérage de la présence ou non d'un ou plusieurs ADN génomiques dans un vecteur de type cosmide ou YAC par exemple.

L'emploi de méthodes plus résolutives peut permettre une mesure supplémentaire de la taille des sondes (champ proche, microscopie électronique, etc.), mais une mesure de l'intensité de fluorescence, s'il s'agit du mode d'observation choisi, peut également fournir cette information.

La méthode que nous decrivons permet de minimiser le nombre d'hybridations nécessaires à l'ordonnancement d'un nombre donné de clones, étant donné un nombre fixé de couleurs de révélation des hybridations, on (plus généralement) de mode distincts de révélation des hybridations.

Légendes des figures

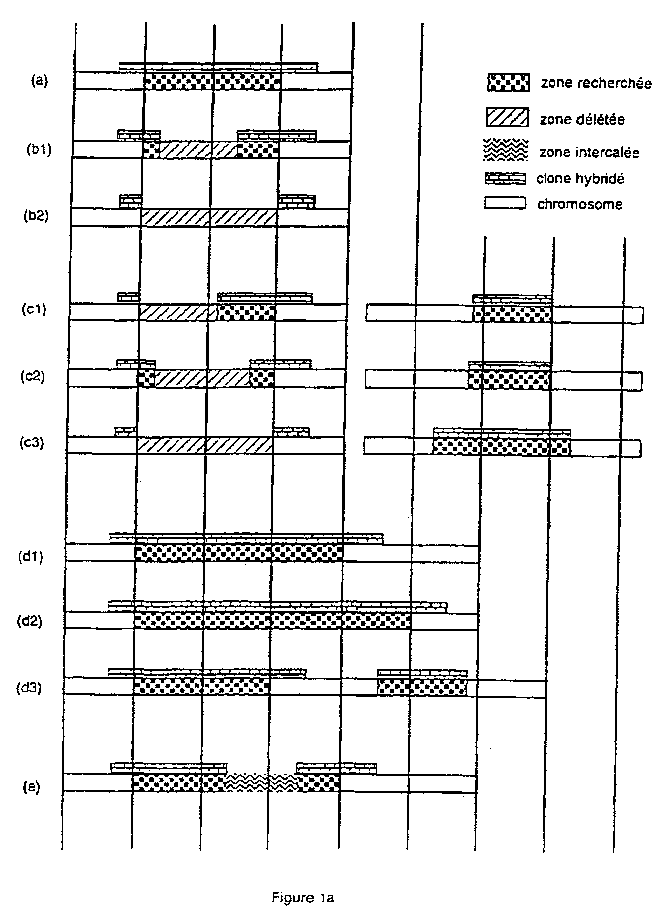

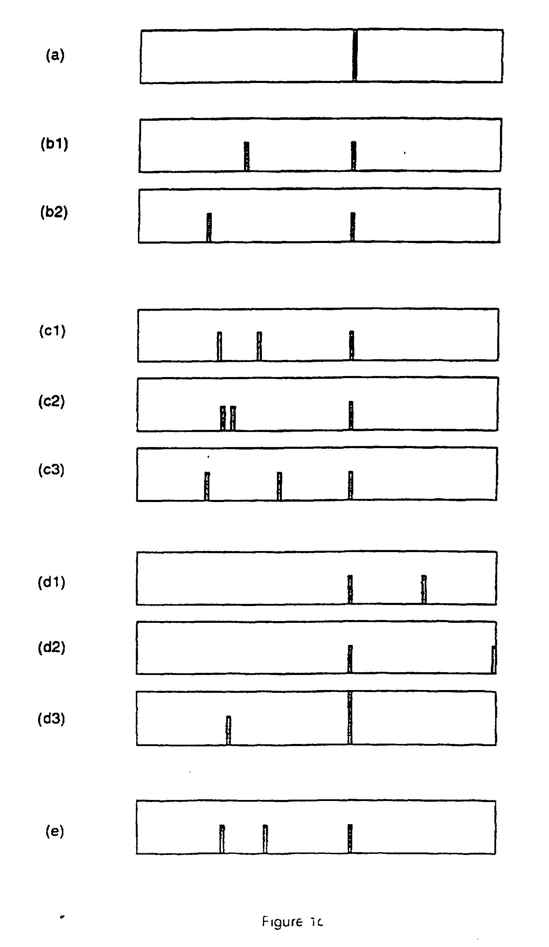

Figure 1a: Illustration du cas d'un clone (rouge) chevauchant le gène (et donc le point de cassure) recherché. Les zones hachurées doivent être imaginées comme absentes, ce qui implique notamment que lorsque deux segments de sondes se trouvent de part et d'autre, ils ne font en fait qu'un segment d'une longueur égale à la somme de leurs longueurs. Le(s) chromosome(s) sousjacents sont représentés par des barres blanches.

(a) situation chez un patient sain.
(b1) délétion(s) d'une partie du gène. (b2) délétion de la totalité du gène.

(c1) et (c2) translocation d'une partie du gène.
(c3) translocation de la totalité du gène.
(d1) duplication(s) d'une partie du gène (éventuellement avec inversion). (d2) duplication(s) de la totalité du gène (éventuellement avec inversion). (d3) répétition(s) d'une partie du gène dans une autre partie du génome. (e) insertion(s) dans le gène, d'une partie différente du gène (et du génome cloné).

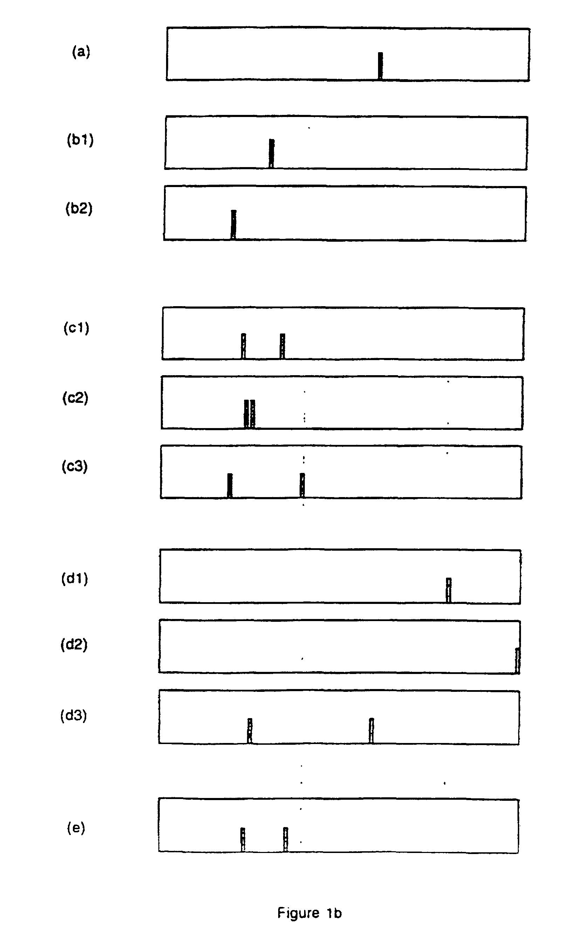

Figure 1b: Histogrammes idéaux des longueurs de clones hybridés correspondant aux situations précédentes. Dans tous les cas (un ou deux pics), la situation est clairement distinguable de celle du clone entier (a).

Les histogrammes de cette figure correspondent aux signaux de l'allèle anormal et sont donc obtenus après soustraction de la contribution due à l'allèle normal, figurée en (a), des histogrammes bruts, représentés sur la figure 1c. En abscisse figure la taille des fragments hybridés, l'échelle (arbitraire) reprenant celle de la figure 1a. En ordonnée figure le nombre de fragments observés, en unité arbitraire, seules important les proportions entre les différentes populations.

Figure 1c: Histogrammes idéaux des longueurs de clones hybridés correspondant aux situations précédentes et tenant compte de l'allèle normal. La contribution de l'allèle normal est représentée par le pic correspondant à la position du cas normal représenté en

(a). Lorsque l'allèle anormal contribue à augmenter la valeur de ce pic (cas (d3)), la contribution de l'allèle normal peut être en général estimée équivalente à la valeur d'un pic anormal.

Figure 2a: Illustration du cas d'un clone chevauchant partiellement le gène recherché.

(a) situation chez un patient sain.
(b1) délétion(s) d'une partie du gène. Une autre situation n'a pas été représentée, où la partie délétée est incluse entièrement dans le clone (cf cas correspondant du schéma 1 a).
(b2) délétion de la totalité du gène.
(c1) et (c2) translocation(s) d'une partie du gène. Une autre situation, correspondant au cas (c2) du schéma la n'a pas été représentée, où la partie transloquée est entièrement comprise dans le clone.
(c3) translocation(s) de la totalité du gène.
(d1) duplication(s) d'une partie du gène repérée par une double flèche (la partie dupliquée est entièrement incluse dans le clone). (d2) duplication(s) d'une partie du gène avec inversion. (d3) duplication(s) d'une partie du gène (la partie dupliquée est partiellement incluse dans le clone).

(d4) duplication(s) de la totalité du gène (sans inversion).

(d5) répétition(s) d'une partie du gène dans une autre partie du génome.

(e) insertion(s) dans le gène d'une partie différente du gène (et du génome cloné).

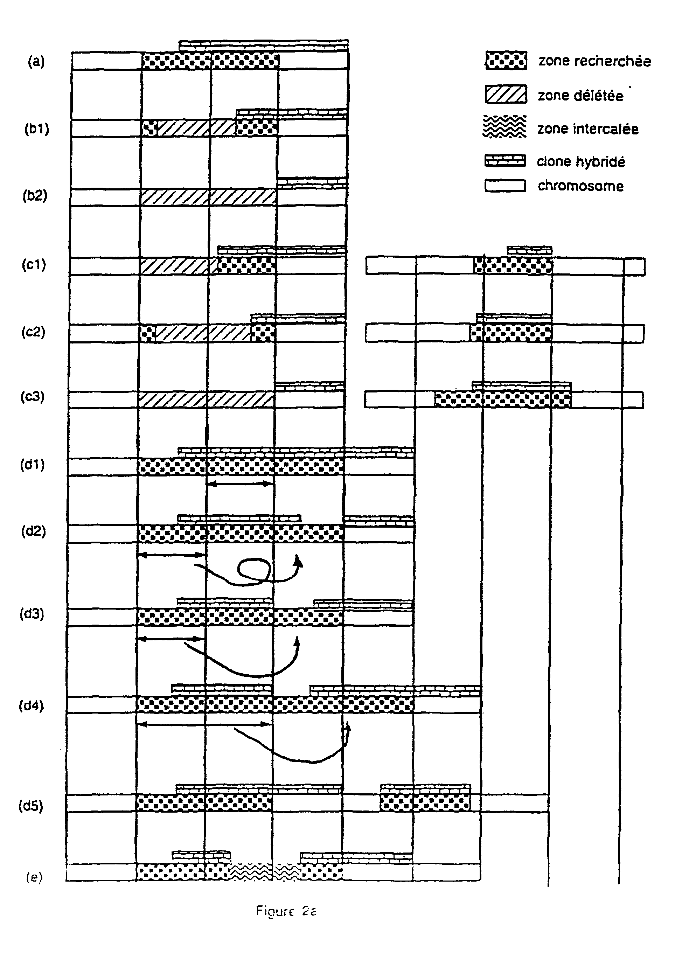

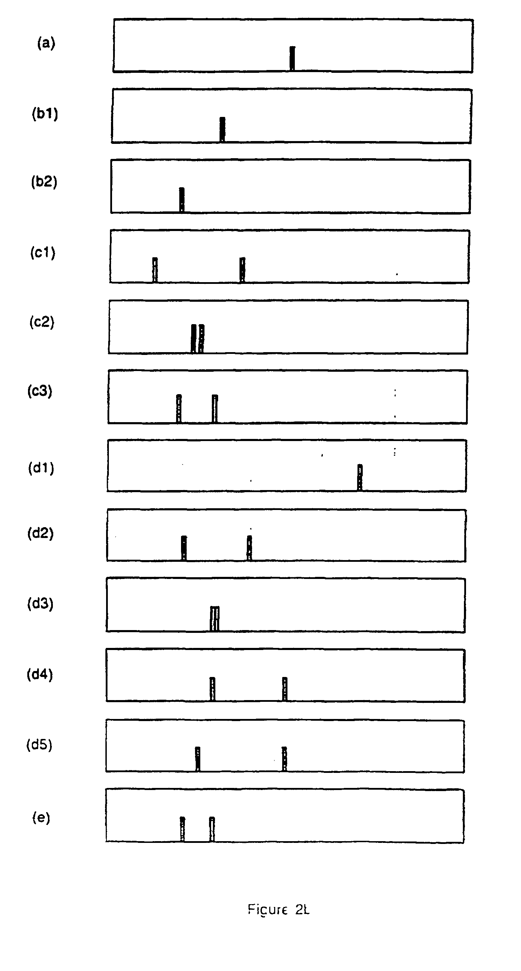

Figure 2b: Histogrammes idéaux des longueurs de clones hybridés correspondant aux situations précédentes. Dans tous les cas (un ou deux pics), la situation est clairement distinguable de celle du clone entier (a).

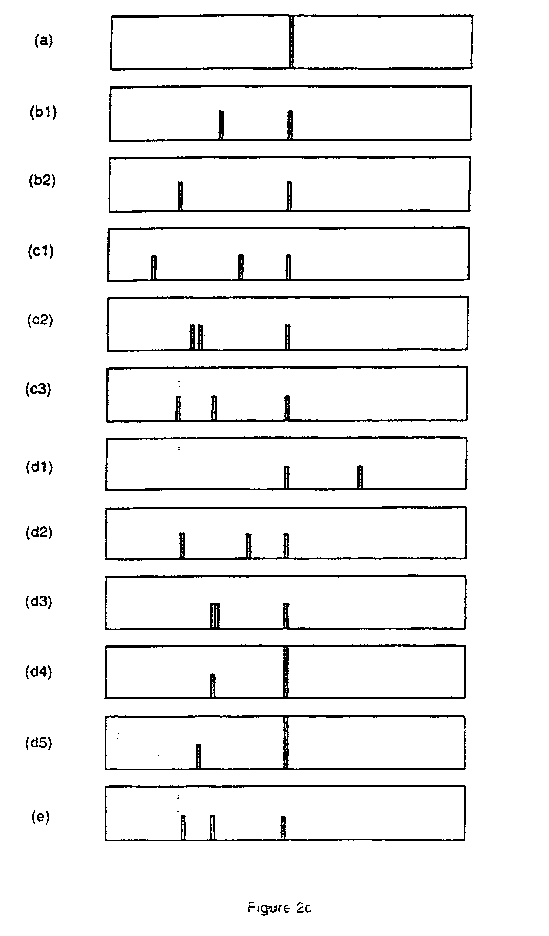

Les histogrammes de cette figure correspondent aux signaux de l'allèle anormal et sont donc obtenus après soustraction de la contribution due à l'allèle normal, figurée en (a), des histogrammes bruts, représentés sur la figure 2c. En abscisse figure la taille des fragments hybridés, l'échelle (arbitraire) reprenant celle de la figure 2a. En ordonnée figure le nombre de fragments observés, en unité arbitraire, seules importent les proportions entre les différentes populations.

Figure 2c: Histogrammes idéaux des longueurs de clones hybridés correspondant aux situations précédentes et tenant compte de l'allèle normal. La contribution de l'allèle normal est représentée par le pic correspondant à la position du cas normal représenté en

(a). Lorsque l'allèle anormal contribue à augmenter la valeur de ce pic (cas (d4) et (d5)), la contribution de l'allèle normal peut être en général estimée équivalente à la valeur d'un pic anormal.

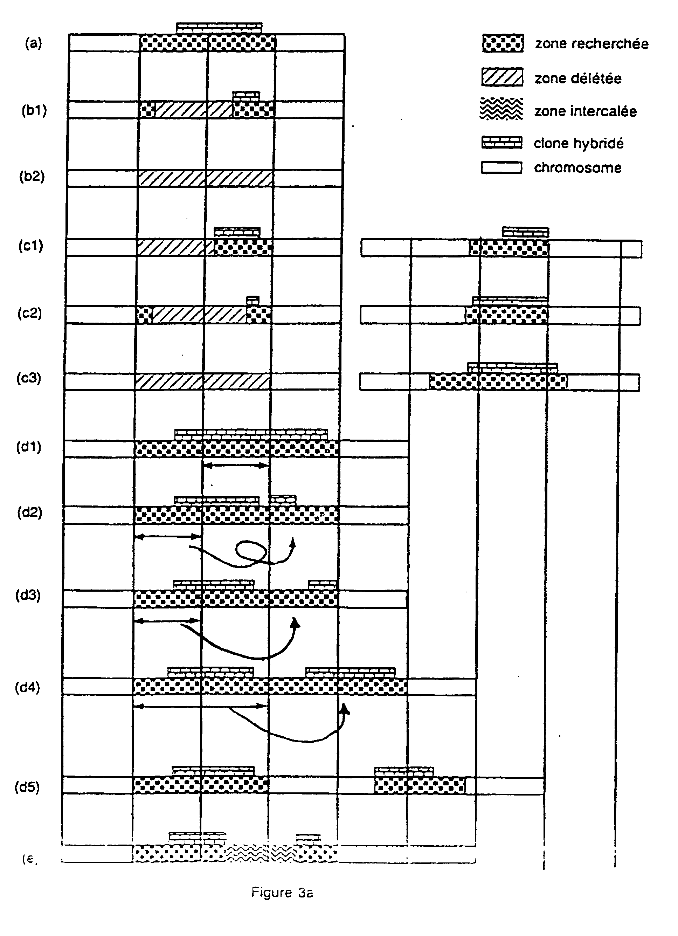

Figure 3a: Illustration du cas d'un clone inclus totalement dans le gène recherché.

(a) situation chez un patient sain.

(b1) délétion(s) d'une partie du gène. Une autre situation n'a pas été représentée, où la partie délétée est incluse entièrement dans le clone (cf cas correspondant du schéma la).

(b2) délétion de la totalité du gène.

(c1) et (c2) translocation(s) d'une partie du gène. Une autre situation, correspondant au cas (c2) du schéma la n'a pas été représentée, où la partie transloquée est entièrement comprise dans le clone.

(c3) translocation(s) de la totalité du gène.

(d1) duplication(s) d'une partie du gène repérée par une double flèche (la partie dupliquée est entièrement incluse dans le clone). (d2) duplication(s) d'une partie du gène avec inversion. (d3) duplication(s) d'une partie du gène (la partie dupliquée est partiellement incluse dans le clone).

(d4) duplication(s) de la totalité du gène (sans inversion).

(d5) répétition(s) d'une partie du gène dans une autre partie du génome.

(e) insertion(s) dans le gène d'une partie différente du gène (et du génome cloné).

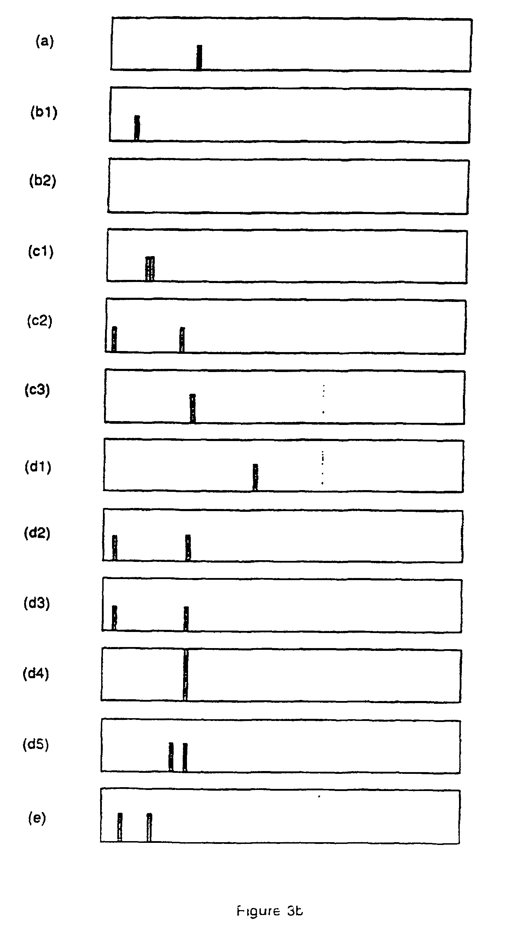

Figure 3b: Histogrammes idéaux des longueurs de clones hybridés correspondant aux situations précédentes. Dans tous les cas (un ou deux pics), la situation est clairement distinguable de celle du clone entier (a).

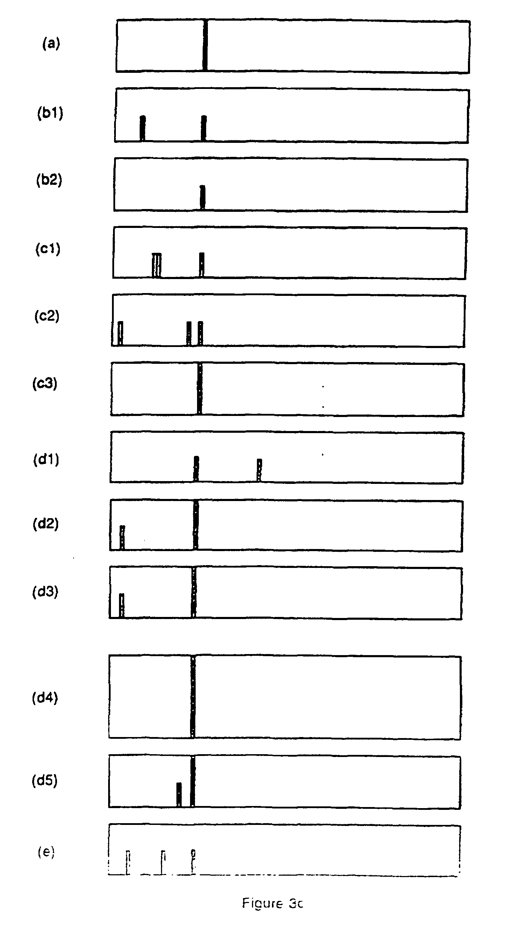

Les histogrammes de cette figure correspondent aux signaux de l'atléle anormal et sont donc obtenus après soustraction de la contribution due à l'allèle normal, figurée en (a), des histogrammes bruts, représentés sur la figure 3c. En abscisse figure la taille des fragments hybridés, l'échelle (arbitraire) reprenant celle de la figure 3a. En ordonnée figure le nombre de fragments observés, en unité arbitraire, seules important les proportions entre les différentes populations.

Figure 3c: Histogrammes idéaux des longueurs de clones hybridés correspondant aux situations précédentes et tenant compte de l'allèle normal. La contribution de l'allèle normal est représentée par le pic correspondant à la position du cas normal représenté en

(a). Lorsque l'allèle anormal contribue à augmenter la valeur de ce pic (cas (c3), (d2), (d3) et (d5)), la contribution de l'allèle normal peut être en général estimée équivalente à la valeur d'un pic anormal.

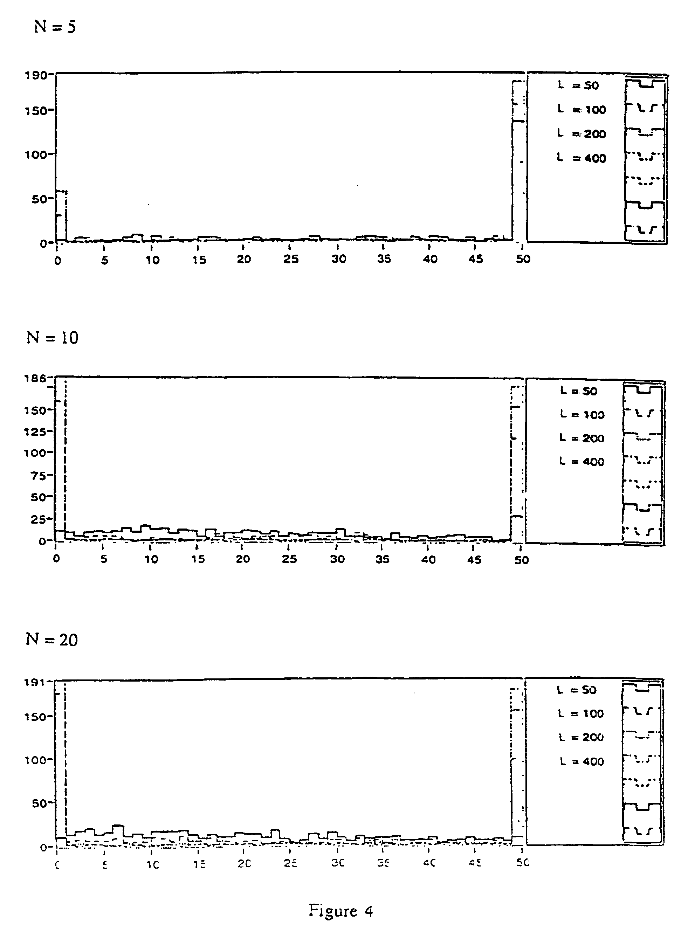

Figure 4: Histogrammes des longueurs du modèle en escalier. Etude de l'influence de la taille caractéristique.

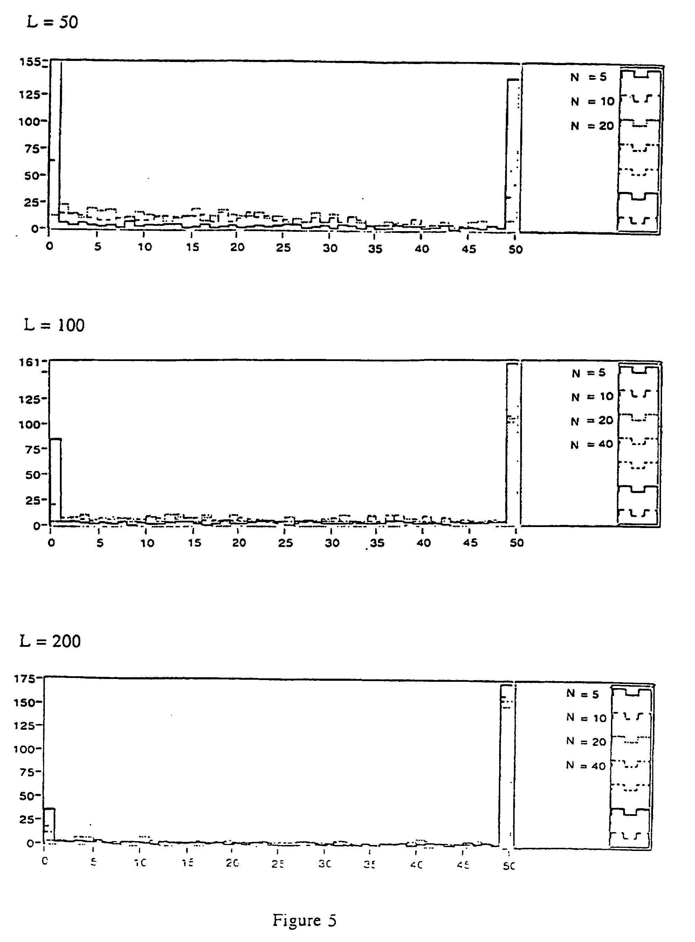

Figure 5: Histogrammes des longueurs du modèle en escalier. Etude de l'influence du taux de cassure.

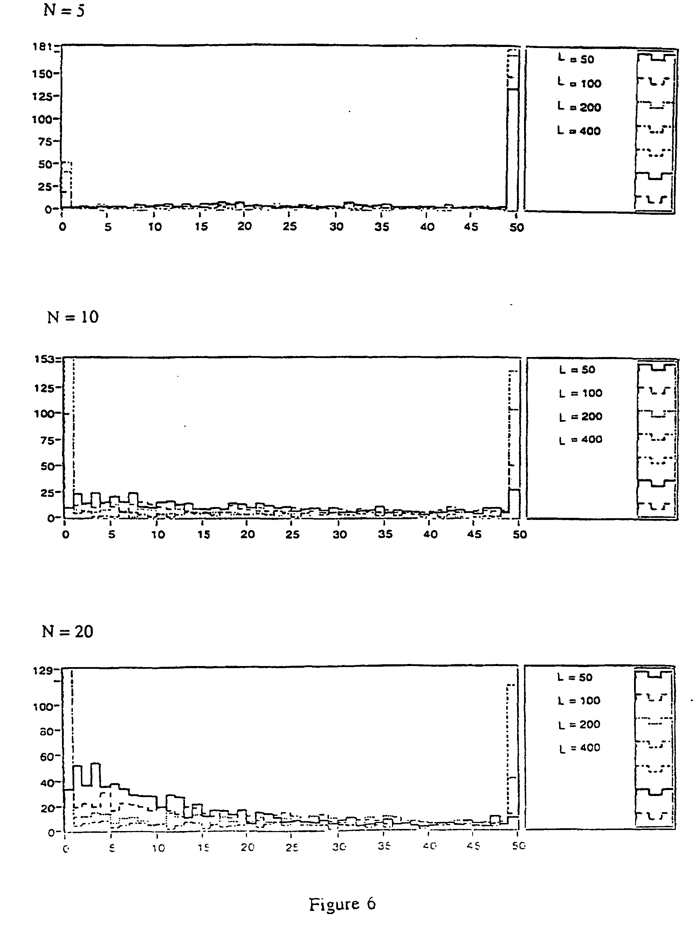

Figure 6: Histogrammes des longueurs du modèle gaussien. Etude de l'influence de la taille caractéristique.

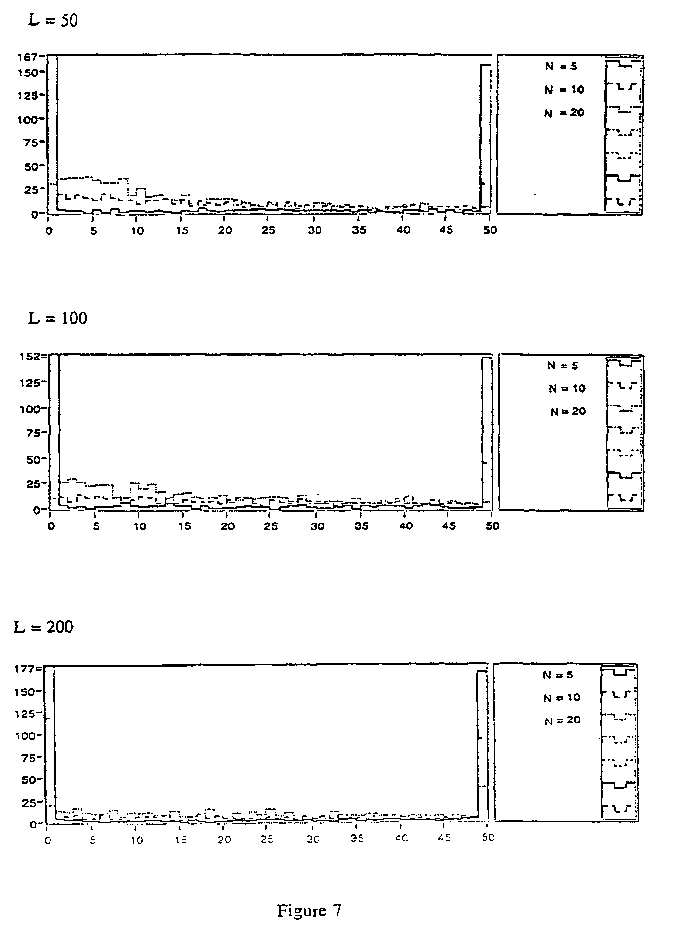

Figure 7: Histogrammes des longueurs du modèle gaussien. Etude de l'influence du taux de cassure.

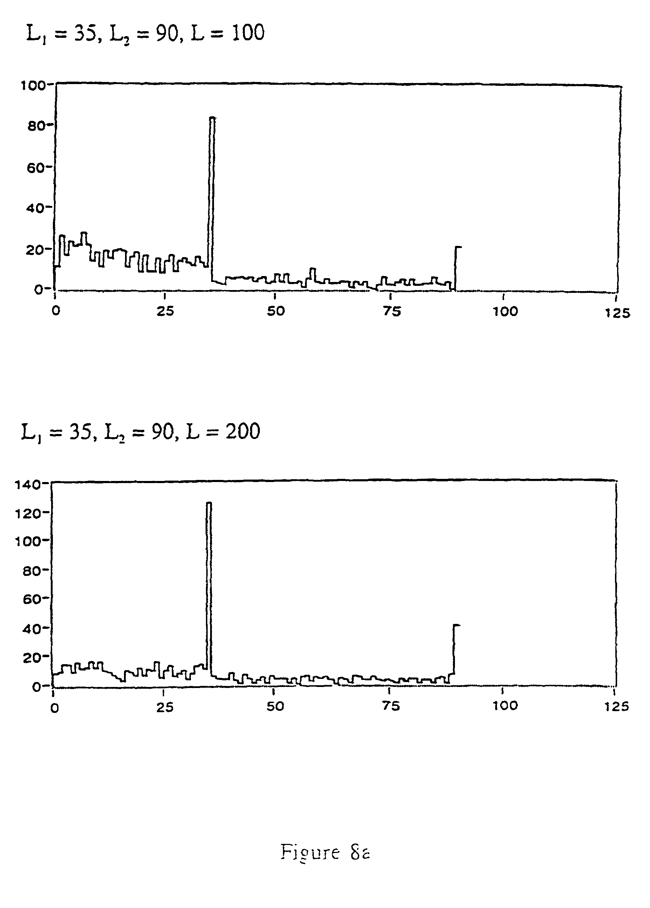

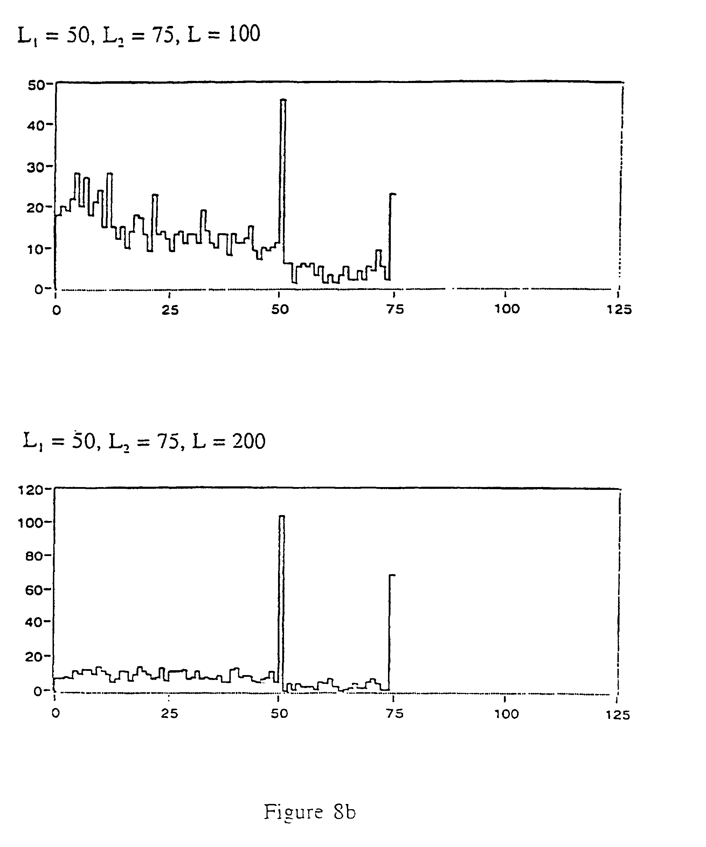

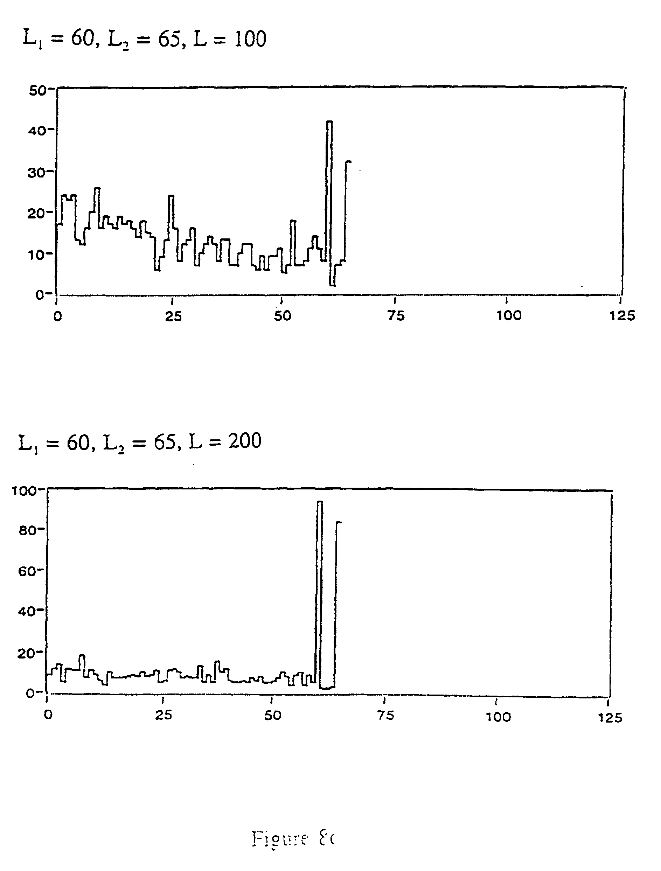

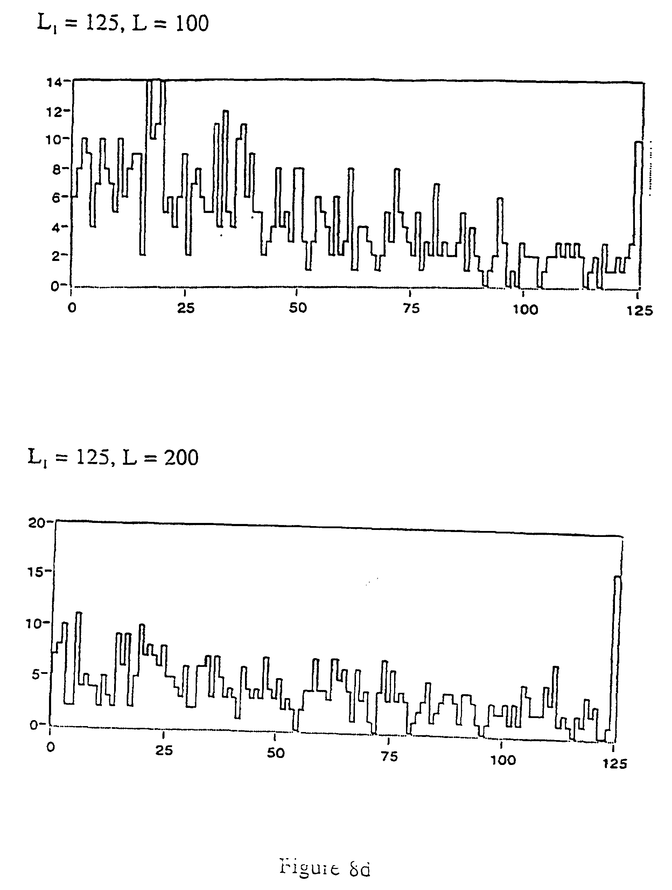

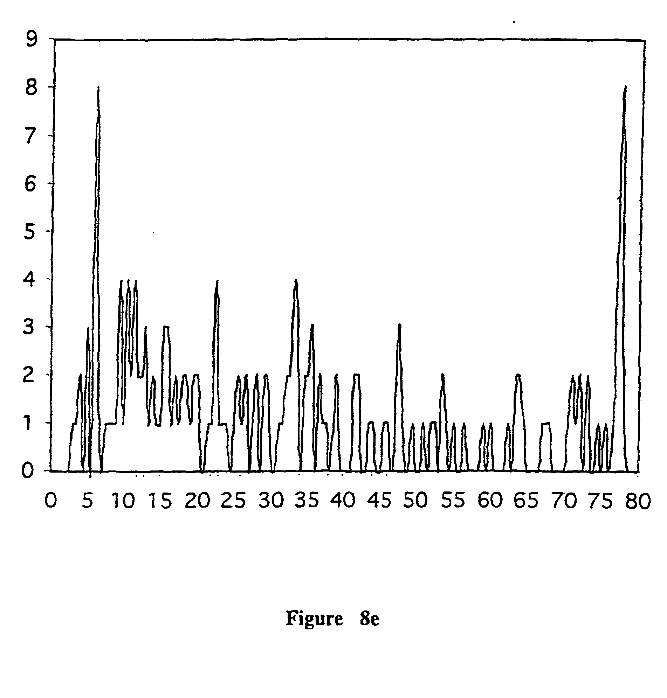

Figure 8: Simulation d'histogrammes dans le cas de l'hybridation d'un BAC (125 kb) sur un point de cassure :

(a) fragments de 35 et 90 kb ; (b) fragments de 50 et 75 kb ; (c) fragments de 60 et 65 kb, (d) situation témoin ; (e) histogramme réel d'un contig de clones cosmidiques hybridés sur de l'ADN génomique humain (longueur du contig : environ 77,5 µm, soit 155 kb). L'axe des abscisses représente la taille des fragments en microns. L'axe des ordonnées représente le nombre de fragments continus de sondes hybridées.

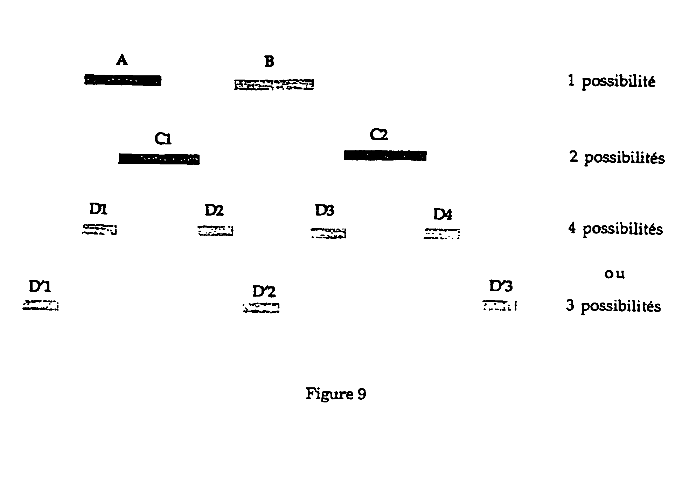

Figure 9: illustration du principe de la cartographie sans codage couleur. Chaque ligne représente le positionnement possible du nouveau sous-clone cartographié par rapport à celui avec lequel il est hybridé. L'orientation des deux premiers clones est arbitraire.

A chaque étape, deux positions de part et d'autre de l'ancien clone utilisé sont possibles pour le nouveau clone hybridé.

Figure 10: illustration du principe de la cartographie avec codage couleur.

A chaque hybridation, les mêmes sous-clones sont hybridés, donnant lieu à un même motif (canevas) d'hybridation si l'on fait abstraction de l'information de couleur. Les deux palettes de couleur utilisées permettent de repérer sans ambiguïté chacun des sous-clones (principe du codage).

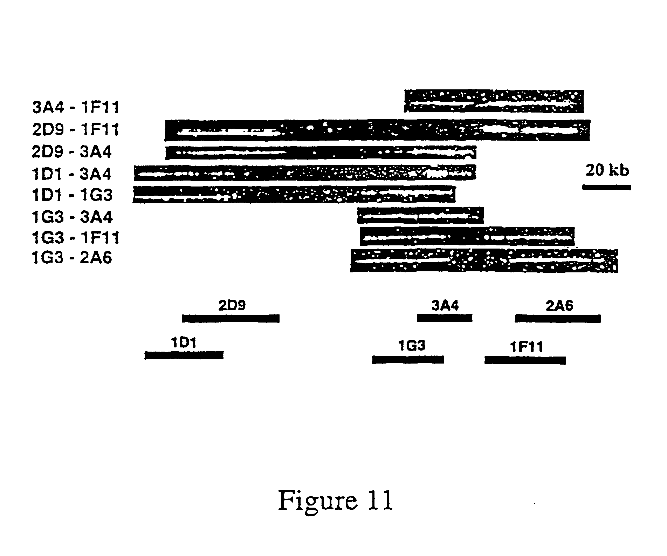

Figure 11 : De l'ADN génomique total de levure contenant un chromosome artificiel (YAC774G4) a été peigné sur des surfaces traitées. Des cosmides appartenant à deux contigs voisins séparés par un intervalle inconnu ont été hybridés deux par deux de façon à pouvoir reconstituer une carte précise de leur disposition. Parmi les cosmides utilisés, 1F11 contient la majeure partie de la séquence du gène de la calpaine 3 dont les mutations sont responsables de la myopathie des ceintures de type 2A.

La figure montre des images caractéristiques de chaque hybridation (un cosmide étant hybridé et révélé en rouge, à gauche, l'autre en vert, à droite) ainsi que la carte reconstituée à partir des mesures, à environ 3 kb près. L'échelle est donnée par la barre de 20 kb. Cette expérience a notamment permis de corriger une carte de la région obtenue à l'aide de STSs (Richard et al., 1995, Mammalian Genome, 6, 754-756) et d'obtenir l'orientation chromosomale du gène.

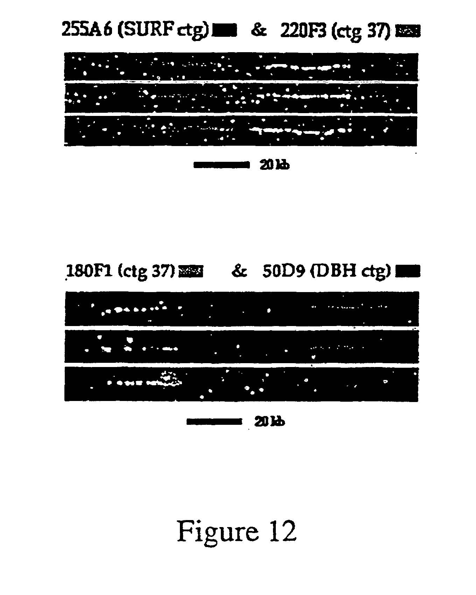

Figure 12 : De l'ADN génomique total humain a été peigné sur des surfaces traitées. Des cosmides appartenant à 3 contigs voisins de la région du chromosome 9 contenant le gène (non cloné) de la "Tuberous Sclerosis 1" (TSCI) ont été hybridés deux par deux et révélés en 2 couleurs différentes afin de mesurer la taille des intervalles séparant ces contigs. La figure montre 3 images caractéristiques de chacune des ces mesures donnant les distances entre cosmides avec une précision de 1,5 à 3 kb pour 50 à 80 mesures. Le code des cosmides utilisés est indiqué, ainsi que le contig auquel ils appartiennent (entre parenthèses). La couleur verte est représentée en blanc et la couleur rouge en gris.

MATERIEL ET METHODES

Méthode des histogrammes

Le schéma de la figure la présente un certain nombre de situations dans le cas d'une sonde recouvrant totalement la région cherchée du génome (région contenant toute séquence génomique dont la modification entraîne l'apparition de la pathologie). De préférence, la sonde recouvre une partie du génome non impliqué dans la cassure, c'est-à-dire de part et d'autre de la délétion par exemple.

Les histogrammes théoriques correspondants (dans l'hypothèse où l'ADN génomique peigné est intact, ce qui est naturellement peu réaliste, et qui sera discuté dans la suite) sont présentés sur le schéma de la figure 1b. La figure 1c représente les résultats bruts avant extraction de la contribution de l'allèle normal. Des situations similaires sont représentées dans le cas où le clone utilisé ne recouvre pas totalement le gène (ou la région du génome) recherché(e) sur les schémas des figures 2a et 3a.

Dans la suite, on utilisera indifféremment l'expression "cette région de génome" (sous-entendu impliquée dans la pathologie) ou "le gène", dans la mesure où la technique ne permet pas de déterminer à proprement parler un gène, mais une région du génome subissant des altérations (se traduisant par un "point de cassure").

L'ensemble des situations représentées sur les schémas précédents donne une idée de la différence attendue entre une situation où la sonde clonée utilisée s'hybride sur une région du génome d'un individu sain qui est modifiée dans le cas d'un individu malade.

Dans tous les cas présentés (sauf celui d'une translocation de tout ou partie du gène contenant la totalité du clone - cf schéma 3a (c3)), l'histogramme (théorique) se distingue de l'histogramme "normal":

- soit par la présence d'un seul pic mais situé à une valeur différente de la longueur du clone (supérieure - cas (d2) par exemple, ou inférieure - cas (b1)).
- soit par la présence de deux pics ou davantage.

La simple donnée de l'histogramme des longueurs et de la longueur Lc du clone (obtenue par exemple à l'aide d'une hybridation sur l'ADN d'un individu sain et obtention du pic de l'histogramme correspondant) permet donc de savoir si le clone étudié recouvre un point de cassure (au sens large entendu ici).

Les anomalies observées au niveau de l'histogramme peuvent être regroupées en plusieurs catégories:

(a) un pic pour une longueur $Li < Lc$
(b) un pic pour une longueur $Ls > Lc$
(c) plusieurs pics de longueur(s) inférieure(s) à $Lc$
(d) plusieurs pics de longueur(s) supérieure(s) à $Lc$

(e) un pic pour une longueur L = Lc et un ou plusieurs pics de longueur(s) inférieure(s) à Lc

(f) un pic pour une longueur L = Lc et un ou plusieurs pics de longueur(s) supérieure(s) à Lc

(g) un ou plusieurs pics de longueurs <, = et > à Lc

Les classes d'anomalies répertoriées précédemment ne suffisent en général malheureusement pas pour définir l'anomalie génétique sous-jacente.

Le schéma de la figure 1b montre en effet qu'il n'est pas possible a priori de distinguer entre une translocation partielle (c1) ou (c2), totale (c3) ou une insertion dans le cas d'un clone qui couvrirait la totalité du gène. Il en est de même pour le cas où le clone ne couvrirait que partiellement le gène (schéma des figures 2b et 3b).

Par contre, la donnée des longueurs correspondant aux pics de l'histogramme permet de préciser dans certains cas la position dans le clone de l'extrémité de la partie du gène délétée, transloquée, dupliquée ou en contact avec une insertion.

En tout état de cause, la mesure d'un histogramme distinct de celui attendu dans le cas d'un génome normal signale la présence dans le génome étudié, d'une anomalie au niveau de la séquence contenue dans le clone utilisé.

(a un pic pour une longueur Li < Lc): il s'agit presque certainement d'une (ou de plusieurs) délétion (s). Cependant, comme nous l'avons noté, il peut s'agir d'un cas où plusieurs pics se trouvent confondus. Nous verrons qu'il est en général possible de distinguer entre ces deux cas par comparaison avec une sonde correspondant à une zone différente du génome (sonde de référence).

Dans le cas d'une délétion, il est possible d'en déduire la taille par soustraction des deux longueurs restantes de la longueur du clone intact (uniquement dans le cas où le clone recouvre la totalité de la partie délétée du gène). La position par rapport au clone ne peut par contre pas être connue, seules des hybridations supplémentaires avec d'autres clones ou des sous-clones pouvant permettre cette détermination.

Cependant le même type d'histogramme peut correspondre à plusieurs délétions, auquel cas il n'est possible que de mesurer la taille totale des délétions (toujours dans l'hypothèse où le clone recouvrirait la totalité de la partie délétée du gène).

Dans les cas où le clone ne recouvre que partiellement le gène, la délétion détectée peut ne représenter qu'une partie de la délétion effective du gène (cf schéma 2a (b1) par exemple).

(b: un pic pour une longueur Ls > Lc): dans ce cas, il s'agit d'une ou de plusieurs duplication(s) à l'intérieur du gène ou à ses extrémités, d'une partie ou de la totalité de celui-ci. Le clone peut recouvrir totalement ou partiellement le gène. Il peut éventuellement y avoir inversion des séquences, mais ceci ne peut être détecté dans le cas où le clone recouvre totalement le gène, La position de la ou des duplications ne peut être déterminée.

née.

(c: plusieurs pics de longueur(s) inférieure(s) à Lc): il peut s'agir dans ce cas de translocations partielles du gène ou de l'insertion d'une séquence à l'intérieur du gène. Dans l'hypothèse d'une translocation, il est possible de préciser les deux extrémités du gène au sein du clone (cas où le clone recouvre totalement le gène), ou d'avancer deux possibilités pour ces extrémités (dans le cas où le clone recouvre partiellement le gène).

(d: plusieurs pics de longueur(s) supérieure(s) à Lc): il s'agit dans ce cas de plusieurs duplication de tout ou partie du gène dans la région du génome couverte par le clone.

(e: un pic pour une longueur L = Lc et un ou plusieurs pics de longueur(s) inférieure(s) à Lc): il s'agit d'une (ou de plusieurs) duplication(s) de tout ou partie du gène dans une zone du génome distincte de la zone couverte par le clone, ceci dans le cas où le clone couvre la totalité ou un partie du gène.

(f: un pic pour une longueur L = Lc et un ou plusieurs pics de longueur(s) supérieure(s) à Lc): bien que cette situation n'ait pas été représentée dans les schémas 1 à 3, il s'agit d'un cas de duplication du type (d) associé à un gène normal.

(g: un ou plusieurs pics de longueurs <, = et > à Lc): combinaisons de (e) et (f).

Dans un certain nombre de situations, l'analyse des signaux d'hybridation et de leur distance, dans le cas où ils seraient alignés, devrait par ailleurs fournir des informations supplémentaires.

L'exemple du schéma la (e) illustrera cette idée: dans ce cas d'une insertion, il faut imaginer que tout segment non hybridé, mais entouré par deux hybridations colinéaires (de tailles quelconques) est susceptible de représenter une insertion: on construira donc un histogramme de ces mesures: s'il existe réellement une insertion, il devrait donc apparaître comme un pic, au même titre que n'importe quelle hybridation.

Cette mesure servira également dans le cas de délétions ou de translocations, par exemple.

La technique exposée précédemment suppose que l'ADN génomique peigné hybridé soit intact. En effet, la situation de référence, celle d'un individu sain dont l'ADN correspondant au clone utilisé pour l'hybridation n'est pas modifié, est représentée théoriquement par un pic à la valeur de la longueur du clone.

Dans la pratique, l'ADN est soumis à des contraintes hydro-mécaniques lors de sa préparation, et également lors du peignage, ce qui conduit à de nombreuses cassures aléatoires. Ces cassures aléatoires peuvent avoir lieu au sein de la séquence cible de la sonde, ce qui donnera lieu, après peignage, à une séparation en plusieurs morceaux de ladite séquence. Lors de l'hybridation avec la sonde, ces segments seront hybridés, et après révélation, mesurés comme des fragments de taille inférieure à celle de la séquence cible.

Ces cassures n'affecteront pas nécessairement l'ensemble des séquences cibles dans l'ensemble des génomes utilisés pour le peignage. En fonction du nombre de génomes humains peignés sur la surface étudiée, et en fonction des conditions de préparation, la proportion des séquences cibles cassées restera raisonnablement faible. Dans ce cas, l'histogramme idéal constitué par un seul pic à la longueur de la sonde sera remplacé par un pic moins important accompagné d'une queue de distribution aux longueurs inférieures.

L'expérience et des considérations pratiques élémentaires montrent qu'un nombre optimal de génomes peignés par surface étudiée est d'au moins une dizaine par surface peignée dans les conditions expérimentales décrites.

Dans ce qui suit, on étudiera des simulations de processus de cassure (consécutives à la préparation et au peignage de l'ADN) dépendant de deux paramètres, permettant d'évaluer l'influence sur l'histogramme théorique des longueurs de ces phénomènes.

Deux paramètres semblent importants dans le phénomène de cassure: la taille caractéristique et le taux de cassure.

La taille caractéristique définit approximativement la taille limite atteinte par des molécules soumises à un grand nombre de manipulations. Un exemple de manipulation possible est le pipetage: en fonction du diamètre du cône de la pipette, la taille limite maximale des molécules sera plus ou moins importante. Un autre exemple de manipulation est le vortexage de la solution d'ADN.

Le taux de cassure est sensé modéliser le nombre de manipulations: par exemple, plus le nombre de pipetage augmente, plus les molécules risquent d'être cassées.

A. Modèle en marche d'escalier.

La façon la plus simple de modéliser des manipulations d'ADN en solution consiste à attribuer une probabilité de cassure nulle aux fragments de taille inférieure à la taille caractéristique $L_O$, et une probabilité de cassure égale à 1 pour les fragments de taille supérieure à $L_O$. La cassure d'un fragment de taille L s'effectue en outre aléatoirement en deux fragments quelconques:

$$L < L_O => P_O(L) = 0$$

$$L > L_O => P_O(L) = 1$$

De façon à introduire un taux de cassure dans ce modèle, un paramètre fixe le nombre de processus de cassure auxquels est soumise une molécule et ses fragments successifs.

Ce modèle permet de simuler les histogrammes attendus lors d'une hybridation d'une sonde de longueur connue $L_C$ en fonction des paramètres du modèle. Les conditions choisies sont: sonde s'hybridant sur un chromosome de 50 Mb, 200 génomes peignés, $L_C$ = 50 kb.

A.1. Influence de la taille caractéristique sur l'histogramme.

La figure 4 représente, pour différents taux (ou nombre d'étapes) de cassure, l'allure des histogrammes théoriques obtenus pour des valeurs de taille caractéristique ($L_O$, $L_{break}$) d'1, 2, 4 ou 8 fois la taille de la sonde. Il apparaît clairement que plus la taille caractéristique est proche de la taille de la sonde, plus l'histogramme se différencie du pic unique décrit dans la partie 2, la queue de la distribution aux longueurs inférieures à la longueur $L_C$ de la sonde prenant de l'importance, jusqu'à pratiquement faire disparaître le pic pour la valeur attendue $L_C$.

A.2. Influence du taux de cassure sur l'histogramme.

La figure 5 présente, pour différentes tailles caractéristiques, l'évolution de l'histogramme théorique en fonction du taux de cassure, c'est-à-dire le nombre d'étapes de cassure aléatoire (N time steps). Il apparaît naturellement que le pic à la longueur attendue $L_C$ diminue lorsque le taux de cassure augmente.

Dans ce modèle cependant, le pic ne disparaît pas totalement, même lorsque le taux de cassure croît indéfiniment, puisqu'aucune cassure n'a lieu lorsque tous les fragments restant sont de taille inférieure à $L_C$.

A.3. Intérêt du modèle.

Le modèle précédent a pour intérêt principal de fournir un moyen rapide pour évaluer l'allure probable de l'histogramme des longueurs des fragments de sonde hybridés, de façon à pouvoir interpréter les résultats des observations.

B. Modèle gaussien.

Un modèle un peu plus réaliste consiste en un modèle similaire d'étapes successives de cassure, mais avec une loi de cassure différente de la loi "tout ou rien" du modèle en marche d'escalier. Une façon simple d'"'arrondir" cette loi est de remplacer la probabilité précédente par une loi de probabilité de cassure gaussienne:

$$P_G (L) = 1 - e^{-(L/Lo)2}$$

B. 1. Influence de la taille caractéristique sur l'histogramme

La figure 6 représente, pour différents taux de cassure, l'allure des histogrammes théoriques obtenus pour des valeurs de taille caractéristique ($L_O$, $L_{break}$) d'1, 2, 4 ou 8 fois la taille de la sonde, identiques à ceux utilisés dans la simulation effectuée dans le cadre du modèle en marche d'escalier. De la même façon, plus la taille caractéristique est proche de la taille de la sonde, plus l'histogramme se différencie du pic unique décrit dans la partie 2, la queue de la distribution aux longueurs inférieures à la longueur Lc de la sonde prenant de l'importance, jusqu'à pratiquement faire disparaître le pic pour la valeur attendue $L_C$.

B.2. Influence du taux de cassure sur l'histogramme.

La figure 5 présente, pour différentes tailles caractéristiques, l'évolution de l'histogramme théorique en fonction du taux de cassure, c'est-à-dire le nombre d'étapes de cassure aléatoire (N time steps). Il apparaît naturellement que le pic à la longueur attendue $L_C$ diminue lorsque le taux de cassure augmente.

Toutefois ce modèle a un comportement plus réaliste que le précédent, puisque lorsque le taux de cassure croît, le pic correspondant à $L_C$ diminue quelle que soit la valeur da la taille caractéristique, pour disparaître complètement lorsque le taux de cassure croit indéfiniment.

B.3. Intérêt du modèle.

Le modèle gaussien, étant plus réaliste, sera celui que nous utiliserons par la suite pour simuler des histogramme de longueurs de fragments de sonde hybridées.

C. Simulation d'une hybridation sur un point de cassure.

Pour étudier les conditions de l'applicabilité de notre technique, nous présentons la simulation de trois types de situations caractéristiques de l'hybridation d'un clone sur un point de cassure. Nous prendrons pour exemple le cas d'un BAC (Bacterial Artificial Chromosome) de 125 kb recouvrant un point de cassure impliqué dans une translocation (cf par exemple schéma 2a (c1)).

Les simulations présentées sur la figure 8d correspondent à une situation où le clone est hybridé sur un ADN génomique normal: dans ces conditions la plupart des hybridations ont une longueur de $L_C$ = 125 kb, mis à part les fragments cassés à cause des manipulations. Nous avons choisi deux valeurs de la taille caractéristique $L_O$: $L_O$ = 100 (< $L_C$) et $L_C$ = 200 (>$L_C$). Le taux de cassure est unique et égal à N = 10.

Ces histogrammes montrent qu'il est possible de distinguer un pic à 125 kb dans la plupart des cas, même assez défavorables comme dans le cas $L_O$ = 100, N = 10.

C.1. Fragments de 35 et 90 kb.

La figure 8a présente les résultats de la simulation d'un histogramme des longueurs de fragments hybridés dans le cas où le BAC de 125 kb s'hybride en deux parties non connexes du génome, de tailles très différentes (35 et 90 kb), comme dans le cas décrit sur le schéma 2a (cl).

Dans les deux cas ($L_O$ = 100, $L_O$ = 200), les deux pics à 35 et 90 kb sont reconnaissables, même si la situation est nettement plus favorable dans le cas où la taille caractéristique est nettement supérieure au plus grand fragment.

Notons par ailleurs que, même dans le cas d'un échantillonage moindre, qui conduirait à un pic à 90 kb peu distinct, le pic à 35 kb subsisterait, signalant une anomalie par rapport à la taille normale du clone.

C.2. Fragments de 50 et 75 kb.

La figure 8b représente le résultat de simulations dans le cas de deux fragments de taille peu différentes l'une de l'autre. Là encore, les pics correspondants sont nets et distincts l'un de l'autre. Dans ce cas, on peut prévoir qu'avec un échantillonnage moindre, il restera possible d'identifier ces deux pics.

C.3. Fragments de 60 et 65 kb.

La figure 8c représente le résultat de simulations dans le cas de deux fragments de tailles très proches. Dans les deux cas, les pics ressortent nettement de l'histogramme des petits fragments, mais il est à craindre que précision limitée des mesures les confondent en un seul.

Là encore, une telle information suffira cependant pour assurer l'existence d'un point de cassure situé à mi-distance du BAC hybridé.

C.4. Conclusions

Les simulations précédentes montrent que, dans des conditions évitant de trop casser l'ADN avant et pendant le peignage, il est possible de distinguer sans ambiguïté entre une situation témoin où la sonde s'hybride sur un génome humain normal, et une situation où la sonde s'hybride sur un génome modifié par la présence d'un point de cassure.

Les paramètres heuristiques caractérisant le peignage montrent qu'il est toutefois préférable d'avoir une taille caractéristique supérieure ou égale à la longueur maximale de l'hybridation. Celle-ci étant inconnue, un critère simple est donc une taille caractéristique de l'ordre de ou supérieure à la longueur du clone utilisé comme sonde.

Pour juger de façon expérimentale de la réalisation de ce critère, il paraît donc souhaitable d'utiliser, en plus des sondes destinées à la recherche d'une anomalie dans le génome, une sonde témoin de taille comparable s'hybridant sur une partie différente du génome. L'histogramme correspondant permettrait ainsi de déterminer qualitativement les paramètres du modèle reflétant la situation expérimentale.

La figure 8e illustre une réalisation expérimentale de l'exemple précédemment décrit dans la figure 8a. De l'ADN génomique humain a été peigné suivant le protocole décrit ci-après. Cinq sondes cosmidiques contiguës du chromosome 9 (280A6, 37A1, 149B8, 134A11, 99B4 appartenant au contig du gène TSCI) totalisant environ 155 kb ont été hybridées sur cet ADN, puis révélées avec des anticorps fluorescents. Un histogramme des longueurs d'hybridation mesurées a été tracé qui est très comparable à l'histogramme de la figure 8d pour $L_{break}$ = 100. On note par ailleurs que l'histogramme réel a été obtenu sur un équivalent de moins de 40 génomes diploïdes (les simulations correspondant à 100 génomes diploïdes).

## Traitement de surface

Des lames de 22 x 22 mm sont préparées et recouvertes de silane en utilisant le procédé décrit dans les demandes de brevet WO 95/22056 et WO 95/21939.

## Préparation des sondes cosmides

L'ADN cosmidique est marqué par une extension à partir d'une amorce statistique avec des nucléotides modifiés par la digoxygénine ou qui sont biotinylés. Pour les sondes marquées à la biotine, on utilise le "kit" de marquage ADN Bioprime™ (Gibco-BRL) contenant des amorces octamères statistiques et de la biotine-14-dCTP. Pour le marquage à la digoxigénine, un mélange différent de dNTPs est utilisé qui contient dCTP, dATP et dGTP (0,1mM), dTTP (0,065 mM) et dig-11-dUTP (0,033 mM).

La taille et la concentration des fragments d'ADN marqués sont confirmées par électrophorèse sur gel d'agarose à 0,6% et par densitométrie de bandes. L'efficacité du marquage est évaluée à partir de taches déposées sur des membranes de nylon en utilisant des dilutions successives de sondes digoxigénine et de sondes biotine. Après incubation avec une phosphatase alcaline anti-dig (AP) (Boehringer Mannheim) ou streptavidine AP (Gibco-BRL), les taches sont révélées avec N BT (NitroBlue Tetrazolium) et BCIP (5-Bromo-4-Chloro-3-Indolylphosphate) de Gibco-BRL. Les taches positives sont comparées avec les résultats obtenus à partir d'échantillons d'ADN contrôles marqués par dig ou par la biotine.

De façon à limiter le bruit de fond, les souches sont finalement purifiées sur des colonnes Bio-Spin 6 (Biorad) par centrifugation 5 mn à 1500 g.

## Solution d'ADN de levure

L'ADN de levure contenant le YAC 774G4 (1600 kb) est préparé dans des blocs de 100 µl d'agarose LMP 0,8 % (1 µg/bloc) en utilisant un protocole de préparation des blocs standard PFGE. Les blocs sont stockés dans l'EDTA 0,5 M à 4°C et rincés avec un tampon TE 15 ml (Tris 10 mM / EDTA 1mM, pH 8) pendant 2 heures avant d'être utilisés. Chaque bloc est coloré avec 3,3 µM YOYO-I (Molecular Probes) dans 100 µl $T_{40}E_2$ (Tris 40 mM / EDTA 2 mM, pH 8) pendant 1 heure à température ambiante (RT). L'agarose est alors fondu pendant 45 minutes à 68°C et digéré 2 heures à 40°C en utilisant 2 U de β-agarase 1 dans un tampon NEB x 1 agarase (Biolabs) par bloc.

La dilution de l'ADN (0,25 µg/ml) dans MES 50 mM (pH 5,5) est effectuée très prudemment de façon à éviter le bris des brins d'ADN. La solution est alors versée dans un réservoir de 4 ml de Teflon™ permettant l'introduction de 3 lames silanisées 22x22 mm pour peignage. La solution d'ADN peut également être stockée à 4°C pendant plusieurs jours.

## Peignage moléculaire

Les lames silanisées sont trempées dans le réservoir de Teflon™ contenant une solution d'ADN de levure totale (0,25 g/ml dans 50 mM MES, pH 5,5), incubées à RT et extraites du réservoir après 10 minutes d'incubation en utilisant un simple dispositif mécanique. Pendant l'incubation, les molécules d'ADN s'ancrent sur la surface par leurs extrémités. En extrayant la surface du réservoir, ceci a le même effet que l'évaporation prévue dans la "méthode de la goutte", le ménisque se déplace par rapport à la surface et exerce une force de traction constante sur les molécules demeurant dans le réservoir.

Les surfaces couvertes avec l'ADN peigné sont alors observées avec un microscope en épifluorescence de façon à contrôler les caractéristiques du peignage. Un enregistrement des champs représentatifs de vue pour chaque lame est effectué pour le contrôle posthybridation.

Les surfaces sont alors collées sur des lames de microscope (cyanocrylate) et chauffées une nuit à 60°C. Elles peuvent être stockées pendant plusieurs mois si on les protège de l'humidité à -20°C ou à température ambiante. Les surfaces sont alors déshydratées avant la dénaturation en utilisant un bain comportant des concentrations croissantes en éthanol (70 %, 90%, 100%).

## Dénaturation et hybridation

Les surfaces sont dénaturées dans 70 % de formamide désionisé/30 % 2xSSC, pH 7,0) pendant 4 minutes à 70_C et immédiatement immergées 5 minutes dans un bain d'éthanol froid (0°C) à des concentrations croissantes (70 %,90 %, 100 %). Les surfaces sont alors mises à sécher.

50 ng de sondes marquées biotine et 50 ng de sondes marquées digoxygénine sont alors mélangés avec 3 μg d'ADN humain Cot1 et 10 μg d'ADN de sperme de hareng dans 10 μl de tampon d'hybridation (50 % de formamide désionisé / 10 % de dextran sulfate / 2xSSC / 1 % Tween 20, pH 7). Les sondes sont dénaturées pendant 5 minutes à 80°C et immédiatement réfrigérées à 0°C.

10 μl de la solution de sonde sont ajoutés par lame peignée de 22x22 mm puis couverts avec une lame non traitée et scellés avec un polymère type Rubbercement commercialisé par Sanford, USA. L'hybridation est conduite pendant une nuit à 37°C dans une chambre humide (HC).

Révélation avec des sondes fluorescentes

Après hybridation, les lames sont lavées pendant 5 minutes à RT dans 3 bains (50 % formamide désionisé/ 2xSSC, pH 7) et pendant 5minutes à 5' at RT dans 3 bains de 2xSSC. Les lames sont incubées pendant 30 minutes à 37°C (HC) avec 50 μl par lame d'une solution de blocage (1,5% -p/v) de réactif (Boehringer Mannheim) dans 4xSSC / Tween 20 0,05 %, pH7,2).

Les détections des sondes marquées à la biotine et à la digoxigénine sont effectuées simultanément en utilisant le même protocole pour chaque couche de détection, chaque couche d'anticorps étant incubée 30 minutes à 37°C, les lames étant lavées après chaque couche (3 fois 5minutes à RT dans 4xSSC / Tween 20 0,05 %).

Pour les sondes marquées à la biotine, les couches suivantes sont utilisées (50 μl de tampon d'hybridation par lame) : (1) 40 mg/ml d'Avidin-Texas Red (Vector). (2) 5 mg/ml d'anti-avidin de chèvre biotinilée (Vector). (3) 40 mg/ml d'Avidin-Texas Red (Vector).

Pour les sondes marquées à la digoxigénine : (1) 34 mg/ml d'un congugué anti-dig de souris avec FITC (Jackson). (2) 28 mg/ml d'anti-souris d'âne-FITC (Jackson). (3) 30 mg/ml d'anti-lapin de souris-FITC (Jackson).

Après détection, les lames sont rincées rapidement dans PBS x 1 et montées avec un réactif (Vectashield , Vector) avant observation. Les lames peuvent être gardées des mois à 4°C dans l'obscurité.

EXEMPLE 1

Dans le cas du peignage d'ADN génomique humain, on effectue des hybridations simultanées de deux cosmides séparés par un gap de plusieurs dizaines de kb (180F1 et 50D9) : environ 80 signaux couplés (cosmide 1 - gap - cosmide 2) ont pu être mesurés sur une seule lamelle de 22x22 mm, correspondant à une distance totale de 120 kb environ (voir exemple 5).

Ce résultat permet d'assurer qu'il est possible d'observer environ une centaine d'hybridations d'un BAC entier sur un génome humain normal. Cette situation expérimentale est à comparer avec les situations théoriques envisagées dans la figure 8: les histogrammes 8d montrent en effet que pour 100 génomes diploïdes (donc 200 occurences d'une séquence clonée), et avec des paramètres particuliers, environ 10 signaux d'hybridation intacts d'un BAC de 125 kb (cas $L_O = 100$ kb), ou 16 signaux d'hybridation intacts (cas $L_O = 200$ kb) sont attendus. Autrement dit, les paramètres théoriques utilisés dans les simulations sont beaucoup plus pessimistes que les conditions expérimentales que l'on est actuellement capable de réaliser.

L'ensemble de cette analyse théorique et expérimentale permet donc une première validation de la présente invention.

Outre la recherche de points de cassure à l'aide de BACs (ou autres sondes de taille équivalente ou inférieure) évoquée précédemment, il est également envisageable d'utiliser des YACs (disponibles pour la totalité du génome humain, et pour d'autres génomes). Des simulations similaires à celles présentées ci-dessus montrent qu'il est envisageable de détecter l'hybridation d'un YAC de 1600 kb en deux fragments distincts de 400 et 1200kb (par exemple), sur la base de 100 génomes peignés dans des conditions correspondant à $L_O$ = 600, N = 10 dans le modèle gaussien.

Cependant, à la différence des observations de BACs ou de sondes de taille semblable ou inférieure, qui peuvent être réalisées sans difficulté à l'aide d'un microscope à épifluorescence équipé d'un objectif x100 ou x 63 et d'une caméra (taille maximale d'un champ de vue; respectivement 123 μm et 195 μm environ), la mesure de fragments de plusieurs centaines de kb impose l'utilisation d'objectifs de grossissement plus faible, pour lesquels des problèmes de détectabilité des signaux peuvent éventuellement se poser:

x 40: champ de vue de taille maximale 307 μm environ.

x 20: champ de vue de taille maximale 614 μm environ.

L'avantage d'utiliser des sondes de taille aussi importante est évidemment de réduire ie nombre d'hybridations nécessaires pour trouver un clone intéressant.

EXEMPLE 2

Dosage de l'ADN phage-λ dans le génome d'E.Coli

De l'ADN génomique d'E. Coli (lc = 4,7 Mb) contenant un exemplaire du génome du phage λ (lignée 5243, lt = 49 kb) a été peigné sur des surfaces silanisées après avoir été contre-coloré avec une molécule fluorescente (YOYO-1).

La longueur totale d'ADN peigné par champ de vue a été estimée à partir de 30 à 50 champs de vue répartis uniformément sur l'ensemble de chaque surface.

De l'ADN de phage λ marqué à la biotine-dUTP a ensuite été hybridé et révélé à l'aide d'un système d'anticorps couplés à la FITC (vert). La longueur totale d'ADN hybridée par champ de vue a été estimée à partir de 100 champs de vue répartis uniformément sur l'ensemble de chaque surface.

Les résultats de 4 expériences sont représentés dans le tableau ci-dessous dans lequel Nb E. coli = $\frac{Lc}{lc}$ et Nb = $\frac{LT}{lt}$

|  | Nb E. Coli | Nb λ | R =λ/E. Coli | ΔR |
|---|---|---|---|---|
| 43_4 | 0,19 | 0,19 | 1,0 | 0,6 |
| 43_6 | 0,25 | 0,22 | 0,9 | 0,5 |
| 43_7 | 0,31 | 0,26 | 0,8 | 0,4 |
| 43_8 | 0,20 | 0,24 | 1,2 | 0,7 |
| 43_9 | 0,26 | 0,39 | 1,5 | 0,8 |
| 43_13 | 0,57 | 0,74 | 1,3 | 0,4 |
| 43_14 | 0,61 | 0,56 | 0,9 | 0,3 |

Ces résultats ont montré, hormis pour la lame 43_9, un rapport raisonnablement proche de la valeur attendue de 1. Cependant le principe utilisé, consistant à mesurer l'ensemble des signaux avant hybridation est peu praticable pour des génomes plus grands (l'incertitude ΔR est ici principalement due au faible nombre de génomes d'E. Coli mesurés, de l'ordre de 6 à 24).

EXEMPLE 3

Dosage du nombre d'amplicons dans le génome de cellules de hamster.

L'étude de faisabilité a été étendue à de l'ADN génomique de mammifère. Le système choisi est l'ADN de 2 lignées de fibroplastes du poumon de hamster (lignées 618 et GMA32) contenant respectivement 1 et 2 copies du gène cible AMP1 par génome haploïde.

De l'ADN génomique de cellules fourni en solution a été peigné sur des surfaces silanisées, avec une densité estimée de 100 génomes diploïdes par surface de 22x22 mm.

Une sonde cosmidique (D3S1, ~40 kb) spécifique de la région du gène cible a été marquée avec de la dig-dUTP. Une autre sonde cosmidique (565.5A1, ~40 kb), spécifique d'une région située à environ 1 Mb du gène cible a été utilisée comme témoin, et marquée avec de la biotine-dUTP.

Les sondes ont été hybridées sur l'ADN génomique peigné préalablement dénaturé, et révélées en rouge (sondes biotinilées) et vert (sondes digoxygénées) dans un des cas, et avec des couleurs inversées dans l'autre. Les signaux d'hybridation de chaque couleur ont été mesurés sur un nombre de champs de vue représentatif de la surface.

Dans le cas de la lignée A32, la taille totale obtenue pour la sonde D3S1 (1400 μm environ) représente 70 copies du gène. La taille obtenue sur les mêmes champs de vue pour la sonde témoin (1180 μm environ) représente quant à elle 60 génomes haploïdes. Cette mesure donne donc un rapport cible/témoin de $1,2 \pm 0,3$ compatible avec la présence d'une copie du gène par génome haploïde.

Ces expériences montrent donc la faisabilité du dosage de gène à partir des seuls signaux d'hybridation de sondes cible et témoin, dès lors qu'un nombre suffisant de signaux peut être observé.

EXEMPLE 4

Cartographie par paires de 6 cosmides sur un YAC de 1600 pb contenu dans de l'ADN génomique de levure

Le YAC 774G4 contient un clone d'ADN génomique humain du chromosome 15 contenant le gène de la calpaine (CANP3), dont la mutation est responsable d'une dystrophie des ceintures (LGMD 2A). En collaboration avec le groupe de J. Beckman du Généthon (Evry), nous avons peigné de l'ADN génomique de levure contenu dans des blocs d'agarose Low Melting (1 bloc, 1 μg d'ADN par bloc) sur des surfaces silanisées.

Les six cosmides ont été hybridés deux par deux et la mesure de leurs taille et distance respectives a été effectuée en constituant des histogrammes des tailles et distances. La moyenne et l'écart-type du pic principal de chaque histogramme en a été extrait à l'aide d'un logiciel spécifique. L'écart-type des mesures s'avère être de l'ordre de 2 à 4 kb (figure 11).

EXEMPLE 5

Cartographie de couples de cosmides sur ADN génomique humain

4 cosmides appartenant à 3 contigs voisins séparés par des gaps ont été utilisés dans 2 séries d'hybridations, effectuées en collaboration avec lc groupe de S. Povey du MRC (Londres). Les contigs recouvrent la région du gène TSC 1 impliqué dans une des formes de la tuberous sclerosis (chromosome 9). Les sondes cosmidiques ont été préparées suivant le protocole ci-dessus.

L'ADN génomique utilisé a été extrait de cultures cellulaires et mis en bloc d'agarose Low Melting à raison de $10^6$ cellules par bloc. 3 blocs traités suivant le protocole précédent (réservoir de 4 ml, utilisation d'une molarité finale de MES pH 5,5 de 150 mM), ont été utilisés pour le peignage de l'ADN génomique.

Les cosmides ont été hybridés 2 par 2 sur des lames semblables, un total de plusieurs dizaines de signaux doubles (rouge/vert alignés) par lame étant en moyenne observé. Le même protocole de mesure que précédemment a été observé, donnant lieu à des valeurs finales ayant la même précision que dans l'expérience précédente.

La figure 12 présente quelques images typiques ayant permis la mesure des tailles des 2 gaps étudiés.

EXEMPLE 6

Cartographie de segments de restriction

Cette technique de cartographie est naturellement applicable à tout autre type d'ADN peigné ou de sous-clone. Une extension possible de la technique consisterait par exemple à cartographier non plus des sous-clones d'un clone, mais directement des fragments de restriction de l'ADN peigné (par exemple un clone de type BAC).

Ceci éviterait l'élaboration de sous-clones intermédiaires avant séquençage, la carte physique des fragments de restriction étant obtenue avec suffisamment de précision pour permettre une reconstitution de la séquence finale. L'applicabilité de cette technique repose sur la bonne séparation des bandes de restriction, et la taille suffisante (> 10 kb) des principaux fragments, ainsi que sur le sous-clonage ultérieur de l'ADN de ces bandes (sous-clonage dans des vecteurs de petite taille, après restriction enzymatique supplémentaire, pour le séquençage).

**Revendications**

1. Procédé de positionnement de plusieurs clones issus d'un génome, **caractérisé en ce que** :

   (a) on fixe et on peigne une certaine quantité dudit génome sur une surface de peignage,
   (b) on hybride le produit de peignage avec des sondes marquées avec des éléments radioactifs, fluorescents ou autres, tels que billes, particules, correspondant à chaque clone, de sorte que lesdites sondes pourront être révélées spécifiquement,
   (c) on prélève l'information correspondant à la place de chaque clone ainsi que les tailles et les distances correspondantes sur le génome,
   (d) on réitère les opérations b) et c) n fois en modifiant la couleur, le marquage ou le mode de révélation des sondes, sachant qu'avec p couleurs, marquages ou modes de révélation différents, au bout de n hybridations on peut positionner $p^n$ clones.

2. Procédé selon la revendication 1, **caractérisé en ce qu'**on utilise des sondes marquées par des nucléotides modifiés qui sont révélés éventuellement de façons distinctes.

3. Procédé selon l'une des revendications 1 à 2, **caractérisé en ce qu'**on utilise des sondes marquées par incorporation de nucléotides modifiés par biotinylation, par DIG ou d'autres haptènes qui sont révélés par un système de couches d'anticorps ou de molécules spécifiques.

4. Procédé selon l'une des revendications 1 à 3, **caractérisé en ce qu'**on utilise aussi des sondes marquées par des nucléotides fluorescents.

5. Procédé selon l'une des revendications 1 à 4, **caractérisé en ce qu'**on dispose sur la surface peignée au moins une dizaine de copies du génome.

6. Procédé selon l'une des revendications 1 à 5, **caractérisé en ce que** la surface comporte un ADN de calibrage qui permet de calibrer chaque mesure.

7. Procédé selon l'une des revendications 1 à 6, **caractérisé en ce que** le génome provient d'un prélèvement de fluide biologique ou d'un tissu d'origine biologique.

8. Procédé selon l'une des revendications 1 à 6, **caractérisé en ce que** le génome provient d'un matériel biologique comportant au moins 80 % de matériel génétique d'origine foetale.

**Claims**

1. Method for positioning several clones originating from a genome, **characterized in that**:

   (a) a certain quantity of said genome is attached to and combed on a combing surface,
   (b) the combing product is hybridized with probes labelled with radioactive or fluorescent elements and the like, such as beads, particles and the like, corresponding to each clone, such that said probes may be specifically revealed,
   (c) the information corresponding to the position of each clone as well as the sizes and the corresponding distances on the genome are extracted,
   (d) operations b) and c) are repeated n times by modifying the colour, the labelling or the mode of revealing the probes, in the knowledge that with p colours, labellings or different modes of revealing, it is possible to position $p^n$ clones after n hybridizations.

2. Method according to Claim 1, **characterized in that** probes labelled with modified nucleotides which are optionally revealed separ.ately are used.

3. Method according to either of Claims 1 and 2, **char-**

**acterized in that** probes labelled by incorporation of nucleotides modified by biotinylation, with DIG or other haptens which are revealed by a system of layers of antibodies or of specific molecules are used.

4. Method according to one of Claims 1 to 3, **characterized in that** probes labelled with fluorescent nucleotides are also used.

5. Method according to one of Claims 1 to 4, **characterized in that** at least about 10 copies of the genome are placed on the combed surface.

6. Method according to one of Claims 1 to 5, **characterized in that** the surface contains a calibrating DNA which makes it possible to calibrate each measurement.

7. Method according to one of Claims 1 to 6, **characterized in that** the genome is obtained from a sample of biological fluid or from a tissue of biological origin.

8. Method according to one of Claims 1 to 6, **characterized in that** the genome is obtained from a biological material containing at least 80% of genetic material of foetal origin.

**Patentansprüche**

1. Verfahren zur Positionsbestimmung von mehreren Klonen, die aus einem Genom hervorgegangen sind, **dadurch gekennzeichnet, dass** man:

(a) eine gewisse Menge des Genoms auf einer Oberfläche zum Kämmen fixiert und kämmt,
(b) das Produkt des Kämmens mit Sonden hybridisiert, die mit radioaktiven, fluoreszierenden oder anderen Elementen markiert sind, wie Kügelchen, Teilchen, welche jedem Klon entsprechen, derart, dass die Sonden spezifisch nachgewiesen werden können,
(c) die Information, die jedem Ort jedes Klons entspricht, sowie die entsprechenden Größen und Entfernungen auf dem Genom entnimmt,
(d) die Vorgehensweisen (b) und (c) n-mal wiederholt, indem man die Farbe, die Markierung oder die Nachweisart der Sonden modifiziert, wobei man weiß, dass man mit p-Farben, Markierungen oder verschiedenen Nachweisarten am Ende von n Hybridisierungen die Position von $p^n$ Klonen bestimmen kann.

2. Verfahren nach Anspruch 1, **dadurch gekennzeichnet, dass** man Sonden verwendet, welche mit modifizierten Nukleotiden markiert sind, die gegebenenfalls auf unterschiedliche Weisen nachgewiesen werden.

3. Verfahren nach einem der Ansprüche 1 bis 2, **dadurch gekennzeichnet, dass** man Sonden verwendet, die durch Einverleibung von Nukleotiden markiert sind, welche durch Biotinylierung, durch DIG oder andere Haptene modifiziert sind, die durch ein System von Schichten von Antikörpern oder spezifischen Molekülen nachgewiesen werden.

4. Verfahren nach einem der Ansprüche 1 bis 3, **dadurch gekennzeichnet, dass** man auch Sonden verwendet, die durch fluoreszierende Nukleotide markiert sind.

5. Verfahren nach einem der Ansprüche 1 bis 4, **dadurch gekennzeichnet, dass** man auf der gekämmten Oberfläche mindestens zehn Kopien des Genoms anordnet.

6. Verfahren nach einem der Ansprüche 1 bis 5, **dadurch gekennzeichnet, dass** die Oberfläche eine Kalibrierungs-DNA umfasst, welche es ermöglicht, jede Messung zu kalibrieren.

7. Verfahren nach einem der Ansprüche 1 bis 6, **dadurch gekennzeichnet, dass** das Genom aus einer Probe von biologischer Flüssigkeit oder einem Gewebe biologischen Ursprungs hervorgeht.

8. Verfahren nach einem der Ansprüche 1 bis 6, **dadurch gekennzeichnet, dass** das Genom aus einem biologischen Material hervorgeht, das mindestens 80% genetisches Material fötalen Ursprungs umfasst.

Figure 1a

(a)

(b1)

(b2)

(c1)

(c2)

(c3)

(d1)

(d2)

(d3)

(e)

Figure 1b

(a)

(b1)

(b2)

(c1)

(c2)

(c3)

(d1)

(d2)

(d3)

(e)

Figure 1c

(a)

(b1)

(b2)

(c1)

(c2)

(c3)

(d1)

(d2)

(d3)

(d4)

(d5)

(e)

zone recherchée

zone délétée

zone intercalée

clone hybridé

chromosome

Figure 2a

(a)

(b1)

(b2)

(c1)

(c2)

(c3)

(d1)

(d2)

(d3)

(d4)

(d5)

(e)

Figure 2L

(a)

(b1)

(b2)

(c1)

(c2)

(c3)

(d1)

(d2)

(d3)

(d4)

(d5)

(e)

Figure 2c

Figure 3a

Figure 3b

Figure 3c

N = 5

N = 10

N = 20

Figure 4

L = 50

L = 100

L = 200

Figure 5

N = 5

N = 10

N = 20

Figure 6

L = 50

L = 100

L = 200

Figure 7

$L_1 = 35, L_2 = 90, L = 100$

$L_1 = 35, L_2 = 90, L = 200$

Figure 8a

$L_1 = 50$, $L_2 = 75$, $L = 100$

$L_1 = 50$, $L_2 = 75$, $L = 200$

Figure 8b

$L_1 = 60, L_2 = 65, L = 100$

$L_1 = 60, L_2 = 65, L = 200$

Figure 8c

$L_1 = 125, L = 100$

$L_1 = 125, L = 200$

Figure 8d

Figure 8e

A     B        1 possibilité

C1       C2    2 possibilités

D1   D2   D3   D4  4 possibilités

ou

D'1    D'2     D'3  3 possibilités

Figure 9

Figure 10

Figure 11

255A6 (SURF ctg)■ & 220F3 (ctg 37)▒

—— 20kb

180F1 (ctg 37)▒ & 50D9 (DBH ctg)■

—— 20kb

Figure 12